# EUROPEAN PATENT APPLICATION

(11) **EP 3 453 401 A1**
(43) Date of publication of application: **13.03.2019**
(21) Application number: 17792510.4
(22) Date of filing: 05.05.2017
(51) Int. Cl.: A61K 38/20, A61P 35/00, C07K 19/00, C12N 15/62, C12N 15/85, C12N 15/86, C12N 15/861, C12N 15/864, C12N 15/867, C12N 5/10

(54) **INTERLEUKIN COMBINATION AND USE THEREOF**

(30) Priority: 06.05.2016 CN 201610297836
(71) Applicant: Wang, Mulin, Shaanxi 719300 (CN); Feng, Huanhuan, Haikou, Hainan 570100 (CN)
(72) Inventor: WANG, Mulin, 719300 Shenmu County Shaanxi (CN); FENG, Huanhuan, 570100 Hainan (CN); ZHNAG, Zhenying, Xiangcheng Henan 466200 (CN); WANG, Linchong, Beijing 100000 (CN); ZHU, Xudong, Tianjin 300000 (CN)
(74) Representative: V.O.
(86) International application number: PCT/CN2017/083203
(87) International publication number: WO 2017/190684

(57) **Abstract**

Disclosed are interleukin combinations or recombinant proteins for preventing and/or treating malignant tumors, and various products prepared with same and the use thereof.

SEQUENCE LISTING

<110>  WANG,Mulin
       FENG,Huanhuan

<120>  INTERLEUKIN COMBINATION AND USE THEREOF

<150>  PCT/CN2017/083203
<151>  2017-05-05

<150>  CN201610297836.1
<151>  2016-05-06

<160>  32

<170>  PatentIn version 3.3

<210>  1
<211>  153
<212>  PRT
<213>  Homo Sapiens

<400>  1

Met Tyr Arg Met Gln Leu Leu Ser Cys Ile Ala Leu Ser Leu Ala Leu
1               5                   10                  15

Val Thr Asn Ser Ala Pro Thr Ser Ser Ser Thr Lys Lys Thr Gln Leu
            20                  25                  30

Gln Leu Glu His Leu Leu Leu Asp Leu Gln Met Ile Leu Asn Gly Ile
        35                  40                  45

Asn Asn Tyr Lys Asn Pro Lys Leu Thr Arg Met Leu Thr Phe Lys Phe
    50                  55                  60

Tyr Met Pro Lys Lys Ala Thr Glu Leu Lys His Leu Gln Cys Leu Glu
65                  70                  75                  80

Glu Glu Leu Lys Pro Leu Glu Glu Val Leu Asn Leu Ala Gln Ser Lys
                85                  90                  95

Asn Phe His Leu Arg Pro Arg Asp Leu Ile Ser Asn Ile Asn Val Ile
            100                 105                 110

Val Leu Glu Leu Lys Gly Ser Glu Thr Thr Phe Met Cys Glu Tyr Ala
        115                 120                 125

Asp Glu Thr Ala Thr Ile Val Glu Phe Leu Asn Arg Trp Ile Thr Phe
    130                 135                 140

Cys Gln Ser Ile Ile Ser Thr Leu Thr
145                 150

<210>  2
<211>  153
<212>  PRT
<213>  Homo Sapiens

<400>  2

<210>  3
<211>  177
<212>  PRT
<213>  Homo Sapiens

<400>  3

Met Phe His Val Ser Phe Arg Tyr Ile Phe Gly Leu Pro Pro Leu Ile
1               5                   10                  15

Leu Val Leu Leu Pro Val Ala Ser Ser Asp Cys Asp Ile Glu Gly Lys
            20                  25                  30

Asp Gly Lys Gln Tyr Glu Ser Val Leu Met Val Ser Ile Asp Gln Leu
        35                  40                  45

Leu Asp Ser Met Lys Glu Ile Gly Ser Asn Cys Leu Asn Asn Glu Phe
    50                  55                  60

Asn Phe Phe Lys Arg His Ile Cys Asp Ala Asn Lys Glu Gly Met Phe
65                  70                  75                  80

Leu Phe Arg Ala Ala Arg Lys Leu Arg Gln Phe Leu Lys Met Asn Ser
                85                  90                  95

Thr Gly Asp Phe Asp Leu His Leu Leu Lys Val Ser Glu Gly Thr Thr
            100                 105                 110

Ile Leu Leu Asn Cys Thr Gly Gln Val Lys Gly Arg Lys Pro Ala Ala
        115                 120                 125

Leu Gly Glu Ala Gln Pro Thr Lys Ser Leu Glu Glu Asn Lys Ser Leu
    130                 135                 140

Lys Glu Gln Lys Lys Leu Asn Asp Leu Cys Phe Leu Lys Arg Leu Leu
145                 150                 155                 160

Gln Glu Ile Lys Thr Cys Trp Asn Lys Ile Leu Met Gly Thr Lys Glu
                165                 170                 175

His

<210>  4
<211>  144
<212>  PRT
<213>  Homo Sapiens

<400>  4

Met Leu Leu Ala Met Val Leu Thr Ser Ala Leu Leu Leu Cys Ser Val
1               5                   10                  15

Ala Gly Gln Gly Cys Pro Thr Leu Ala Gly Ile Leu Asp Ile Asn Phe
            20                  25                  30

Leu Ile Asn Lys Met Gln Glu Asp Pro Ala Ser Lys Cys His Cys Ser
        35                  40                  45

Ala Asn Val Thr Ser Cys Leu Cys Leu Gly Ile Pro Ser Asp Asn Cys
    50                  55                  60

Thr Arg Pro Cys Phe Ser Glu Arg Leu Ser Gln Met Thr Asn Thr Thr
65                  70                  75                  80

Met Gln Thr Arg Tyr Pro Leu Ile Phe Ser Arg Val Lys Lys Ser Val
                85                  90                  95

Glu Val Leu Lys Asn Asn Lys Cys Pro Tyr Phe Ser Cys Glu Gln Pro
            100                 105                 110

Cys Asn Gln Thr Thr Ala Gly Asn Ala Leu Thr Phe Leu Lys Ser Leu
        115                 120                 125

Leu Glu Ile Phe Gln Lys Glu Lys Met Arg Gly Met Arg Gly Lys Ile
    130                 135                 140

<210>  5
<211>  162
<212>  PRT
<213>  Homo Sapiens

<400>  5

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser

<210>  6
<211>  162
<212>  PRT
<213>  Homo Sapiens

<400>  6

Met Arg Ser Ser Pro Gly Asn Met Glu Arg Ile Val Ile Cys Leu Met
1               5                   10                  15

Val Ile Phe Leu Gly Thr Leu Val His Lys Ser Ser Ser Gln Gly Gln
            20                  25                  30

Asp Arg His Met Ile Arg Met Arg Gln Leu Ile Asp Ile Val Asp Gln
        35                  40                  45

Leu Lys Asn Tyr Val Asn Asp Leu Val Pro Glu Phe Leu Pro Ala Pro
    50                  55                  60

Glu Asp Val Glu Thr Asn Cys Glu Trp Ser Ala Phe Ser Cys Phe Gln
65                  70                  75                  80

Lys Ala Gln Leu Lys Ser Ala Asn Thr Gly Asn Asn Glu Arg Ile Ile
                85                  90                  95

Asn Val Ser Ile Lys Lys Leu Lys Arg Lys Pro Pro Ser Thr Asn Ala
            100                 105                 110

Gly Arg Arg Gln Lys His Arg Leu Thr Cys Pro Ser Cys Asp Ser Tyr
        115                 120                 125

Glu Lys Lys Pro Pro Lys Glu Phe Leu Glu Arg Phe Lys Ser Leu Leu
    130                 135                 140

Gln Lys Met Ile His Gln His Leu Ser Ser Arg Thr His Gly Ser Glu
145                 150                 155                 160

Asp Ser

<210>  7
<211>  177
<212>  PRT
<213>  Homo Sapiens

<400>  7

Met Arg Glu Arg Pro Arg Leu Gly Glu Asp Ser Ser Leu Ile Ser Leu
1               5                   10                  15

Phe Leu Gln Val Val Ala Phe Leu Ala Met Val Met Gly Thr His Thr
            20                  25                  30

Tyr Ser His Trp Pro Ser Cys Cys Pro Ser Lys Gly Gln Asp Thr Ser
        35                  40                  45

Glu Glu Leu Leu Arg Trp Ser Thr Val Pro Val Pro Pro Leu Glu Pro
    50                  55                  60

Ala Arg Pro Asn Arg His Pro Glu Ser Cys Arg Ala Ser Glu Asp Gly
65                  70                  75                  80

Pro Leu Asn Ser Arg Ala Ile Ser Pro Trp Arg Tyr Glu Leu Asp Arg
                85                  90                  95

Asp Leu Asn Arg Leu Pro Gln Asp Leu Tyr His Ala Arg Cys Leu Cys
            100                 105                 110

Pro His Cys Val Ser Leu Gln Thr Gly Ser His Met Asp Pro Arg Gly
        115                 120                 125

Asn Ser Glu Leu Leu Tyr His Asn Gln Thr Val Phe Tyr Arg Arg Pro
    130                 135                 140

Cys His Gly Glu Lys Gly Thr His Lys Gly Tyr Cys Leu Glu Arg Arg
145                 150                 155                 160

Leu Tyr Arg Val Ser Leu Ala Cys Val Cys Val Arg Pro Arg Val Met
                165                 170                 175

Gly



<210>  8
<211>  270
<212>  PRT
<213>  Homo Sapiens

<400>  8

Met Lys Pro Lys Met Lys Tyr Ser Thr Asn Lys Ile Ser Thr Ala Lys
1               5                   10                  15

Trp Lys Asn Thr Ala Ser Lys Ala Leu Cys Phe Lys Leu Gly Lys Ser

20                  25                  30

Gln Gln Lys Ala Lys Glu Val Cys Pro Met Tyr Phe Met Lys Leu Arg
        35                  40                  45

Ser Gly Leu Met Ile Lys Lys Glu Ala Cys Tyr Phe Arg Arg Glu Thr
    50                  55                  60

Thr Lys Arg Pro Ser Leu Lys Thr Gly Arg Lys His Lys Arg His Leu
65                  70                  75                  80

Val Leu Ala Ala Cys Gln Gln Gln Ser Thr Val Glu Cys Phe Ala Phe
                85                  90                  95

Gly Ile Ser Gly Val Gln Lys Tyr Thr Arg Ala Leu His Asp Ser Ser
            100                 105                 110

Ile Thr Gly Ile Ser Pro Ile Thr Glu Tyr Leu Ala Ser Leu Ser Thr
        115                 120                 125

Tyr Asn Asp Gln Ser Ile Thr Phe Ala Leu Glu Asp Glu Ser Tyr Glu
    130                 135                 140

Ile Tyr Val Glu Asp Leu Lys Lys Asp Glu Lys Lys Asp Lys Val Leu
145                 150                 155                 160

Leu Ser Tyr Tyr Glu Ser Gln His Pro Ser Asn Glu Ser Gly Asp Gly
                165                 170                 175

Val Asp Gly Lys Met Leu Met Val Thr Leu Ser Pro Thr Lys Asp Phe
            180                 185                 190

Trp Leu His Ala Asn Asn Lys Glu His Ser Val Glu Leu His Lys Cys
        195                 200                 205

Glu Lys Pro Leu Pro Asp Gln Ala Phe Phe Val Leu His Asn Met His
    210                 215                 220

Ser Asn Cys Val Ser Phe Glu Cys Lys Thr Asp Pro Gly Val Phe Ile
225                 230                 235                 240

Gly Val Lys Asp Asn His Leu Ala Leu Ile Lys Val Asp Ser Ser Glu
                245                 250                 255

Asn Leu Cys Thr Glu Asn Ile Leu Phe Lys Leu Ser Glu Thr
            260                 265                 270

<210> 9
<211> 462
<212> DNA
<213> Homo Sapiens

<400> 9
atgtacagga tgcaactcct gtcttgcatt gcactaagtc ttgcacttgt cacaaacagt 60

gcacctactt caagttctac aaagaaaaca cagctacaac tggagcattt actgctggat 120

ttacagatga ttttgaatgg aattaataat tacaagaatc ccaaactcac caggatgctc 180

acatttaagt tttacatgcc caagaaggcc acagaactga aacatcttca gtgtctagaa 240

gaagaactca aacctctgga ggaagtgcta aatttagctc aaagcaaaaa ctttcactta 300

agacccaggg acttaatcag caatatcaac gtaatagttc tggaactaaa gggatctgaa 360

acaacattca tgtgtgaata tgctgatgag acagcaacca ttgtagaatt tctgaacaga 420

tggattacct tttgtcaaag catcatctca acactgactt ga 462

<210> 10
<211> 462
<212> DNA
<213> Homo Sapiens

<400> 10
atgggtctca cctcccaact gcttccccct ctgttcttcc tgctagcatg tgccggcaac 60

tttgtccacg gacacaagtg cgatatcacc ttacaggaga tcatcaaaac tttgaacagc 120

ctcacagagc agaagactct gtgcaccgag ttgaccgtaa cagacatctt tgctgcctcc 180

aagaacacaa ctgagaagga aaccttctgc agggctgcga ctgtgctccg gcagttctac 240

agccaccatg agaaggacac tcgctgcctg ggtgcgactg cacagcagtt ccacaggcac 300

aagcagctga tccgattcct gaaacggctc gacaggaacc tctggggcct ggcgggcttg 360

aattcctgtc ctgtgaagga agccaaccag agtacgttgg aaaacttctt ggaaaggcta 420

aagacgatca tgagagagaa atattcaaag tgttcgagct ga 462

<210> 11
<211> 534
<212> DNA
<213> Homo Sapiens

<400> 11
atgttccatg tttcttttag gtatatcttt ggacttcctc ccctgatcct tgttctgttg 60

ccagtagcat catctgattg tgatattgaa ggtaaagatg gcaaacaata tgagagtgtt 120

ctaatggtca gcatcgatca attattggac agcatgaaag aaattggtag caattgcctg 180

aataatgaat ttaacttttt taaaagacat atctgtgatg ctaataagga aggtatgttt 240

ttattccgtg ctgctcgcaa gttgaggcaa tttcttaaaa tgaatagcac tggtgatttt 300

gatctccact tattaaaagt ttcagaaggc acaacaatac tgttgaactg cactggccag 360

gttaaaggaa gaaaaccagc tgccctgggt gaagcccaac caacaaagag tttggaagaa 420

aataaatctt taaaggaaca gaaaaaactg aatgacttgt gtttcctaaa gagactatta 480

caagagataa aaacttgttg gaataaaatt ttgatgggca ctaaagaaca ctga 534

<210> 12
<211> 435
<212> DNA
<213> Homo Sapiens

<400> 12
atgcttctgg ccatggtcct tacctctgcc ctgctcctgt gctccgtggc aggccagggg 60

tgtccaacct tggcggggat cctggacatc aacttcctca tcaacaagat gcaggaagat 120

ccagcttcca agtgccactg cagtgctaat gtgaccagtt gtctctgttt gggcattccc 180

tctgacaact gcaccagacc atgcttcagt gagagactgt ctcagatgac caataccacc 240

atgcaaacaa gatacccact gattttcagt cgggtgaaaa aatcagttga agtactaaag 300

aacaacaagt gtccatattt ttcctgtgaa cagccatgca accaaaccac ggcaggcaac 360

gcgctgacat ttctgaagag tcttctggaa attttccaga aagaaaagat gagagggatg 420

agaggcaaga tatga 435

<210> 13
<211> 489
<212> DNA
<213> Homo Sapiens

<400> 13
atgagaattt cgaaaccaca tttgagaagt atttccatcc agtgctactt gtgtttactt 60

ctaaacagtc attttctaac tgaagctggc attcatgtct tcattttggg ctgtttcagt 120

gcagggcttc ctaaaacaga agccaactgg gtgaatgtaa taagtgattt gaaaaaaatt 180

gaagatctta ttcaatctat gcatattgat gctactttat atacggaaag tgatgttcac 240

cccagttgca aagtaacagc aatgaagtgc tttctcttgg agttacaagt tatttcactt 300

gagtccggag atgcaagtat tcatgataca gtagaaaatc tgatcatcct agcaaacaac 360

agtttgtctt ctaatgggaa tgtaacagaa tctggatgca aagaatgtga ggaactggag 420

gaaaaaaata ttaaagaatt tttgcagagt tttgtacata ttgtccaaat gttcatcaac 480

acttcttga 489

<210> 14
<211> 489
<212> DNA
<213> Homo Sapiens

<400> 14
atgagatcca gtcctggcaa catggagagg attgtcatct gtctgatggt catcttcttg 60

gggacactgg tccacaaatc aagctcccaa ggtcaagatc gccacatgat tagaatgcgt 120

caacttatag atattgttga tcagctgaaa aattatgtga atgacttggt ccctgaattt 180

ctgccagctc cagaagatgt agagacaaac tgtgagtggt cagctttttc ctgctttcag 240

aaggcccaac taaagtcagc aaatacagga aacaatgaaa ggataatcaa tgtatcaatt 300

aaaaagctga agaggaaacc accttccaca aatgcaggga gaagacagaa acacagacta 360

acatgccctt catgtgattc ttatgagaaa aaaccaccca aagaattcct agaaagattc 420

aaatcacttc tccaaaagat gattcatcag catctgtcct ctagaacaca cggaagtgaa 480

gattcctga 489

<210> 15
<211> 534
<212> DNA
<213> Homo Sapiens

<400> 15
atgagggagc gacccagatt aggtgaggac agttctctca ttagcctttt cctacaggtg 60

gttgcattct tggcaatggt catgggaacc cacacctaca gccactggcc cagctgctgc 120

cccagcaaag ggcaggacac ctctgaggag ctgctgaggt ggagcactgt gcctgtgcct 180

cccctagagc ctgctaggcc caaccgccac ccagagtcct gtagggccag tgaagatgga 240

cccctcaaca gcagggccat ctccccctgg agatatgagt tggacagaga cttgaaccgg 300

ctcccccagg acctgtacca cgcccgttgc ctgtgcccgc actgcgtcag cctacagaca 360

ggctcccaca tggacccccg gggcaactcg gagctgctct accacaacca gactgtcttc 420

taccggcggc catgccatgg cgagaagggc acccacaagg gctactgcct ggagcgcagg 480

ctgtaccgtg tttccttagc ttgtgtgtgt gtgcggcccc gtgtgatggg ctag 534

<210> 16
<211> 813
<212> DNA
<213> Homo Sapiens

<400> 16
atgaagccta aaatgaagta ttcaaccaac aaaatttcca cagcaaagtg gaagaacaca 60

gcaagcaaag ccttgtgttt caagctggga aaatcccaac agaaggccaa agaagtttgc 120

cccatgtact ttatgaagct ccgctctggc cttatgataa aaaaggaggc ctgttacttt 180

aggagagaaa ccaccaaaag gccttcactg aaaacaggta gaaagcacaa aagacatctg 240

gtactcgctg cctgtcaaca gcagtctact gtggagtgct ttgcctttgg tatatcaggg 300

gtccagaaat atactagagc acttcatgat tcaagtatca caggaatttc acctattaca 360

gagtatcttg cttctctaag cacatacaat gatcaatcca ttacttttgc tttggaggat 420

gaaagttatg agatatatgt tgaagacttg aaaaaagatg aaaagaaaga taaggtgtta 480

ctgagttact atgagtctca acacccctca aatgaatcag gtgacggtgt tgatggtaag 540

atgttaatgg taaccctgag tcctacaaaa gacttctggt tgcatgccaa caacaaggaa 600

cactctgtgg agctccataa gtgtgaaaaa ccactgccag accaggcctt ctttgtcctt 660

cataatatgc actccaactg tgtttcattt gaatgcaaga ctgatcctgg agtgtttata 720

ggtgtaaagg ataatcatct tgctctgatt aaagtagact cttctgagaa tttgtgtact 780

gaaaatatct tgtttaagct ctctgaaact tag 813

<210> 17
<211> 752
<212> PRT
<213> Artificial sequence

<400> 17

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala

                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu Gly Gly Gly Gly Ser Gly Gly
            580                 585                 590

Gly Gly Ser Gly Gly Gly Gly Ser Asp Cys Asp Ile Glu Gly Lys Asp
        595                 600                 605

Gly Lys Gln Tyr Glu Ser Val Leu Met Val Ser Ile Asp Gln Leu Leu
    610                 615                 620

Asp Ser Met Lys Glu Ile Gly Ser Asn Cys Leu Asn Asn Glu Phe Asn
625                 630                 635                 640

Phe Phe Lys Arg His Ile Cys Asp Ala Asn Lys Glu Gly Met Phe Leu
                645                 650                 655

Phe Arg Ala Ala Arg Lys Leu Arg Gln Phe Leu Lys Met Asn Ser Thr
            660                 665                 670

Gly Asp Phe Asp Leu His Leu Leu Lys Val Ser Glu Gly Thr Thr Ile
        675                 680                 685

Leu Leu Asn Cys Thr Gly Gln Val Lys Gly Arg Lys Pro Ala Ala Leu
    690                 695                 700

Gly Glu Ala Gln Pro Thr Lys Ser Leu Glu Glu Asn Lys Ser Leu Lys
705                 710                 715                 720

Glu Gln Lys Lys Leu Asn Asp Leu Cys Phe Leu Lys Arg Leu Leu Gln
                725                 730                 735

Glu Ile Lys Thr Cys Trp Asn Lys Ile Leu Met Gly Thr Lys Glu His
            740                 745                 750

<210>  18
<211>  745
<212>  PRT
<213>  Artificial sequence

<400>  18

Tyr Ser His Trp Pro Ser Cys Cys Pro Ser Lys Gly Gln Asp Thr Ser
1               5                   10                  15

Glu Glu Leu Leu Arg Trp Ser Thr Val Pro Val Pro Pro Leu Glu Pro
            20                  25                  30

Ala Arg Pro Asn Arg His Pro Glu Ser Cys Arg Ala Ser Glu Asp Gly
        35                  40                  45

Pro Leu Asn Ser Arg Ala Ile Ser Pro Trp Arg Tyr Glu Leu Asp Arg
    50                  55                  60

Asp Leu Asn Arg Leu Pro Gln Asp Leu Tyr His Ala Arg Cys Leu Cys
65                  70                  75                  80

Pro His Cys Val Ser Leu Gln Thr Gly Ser His Met Asp Pro Arg Gly
                85                  90                  95

Asn Ser Glu Leu Leu Tyr His Asn Gln Thr Val Phe Tyr Arg Arg Pro
            100                 105                 110

Cys His Gly Glu Lys Gly Thr His Lys Gly Tyr Cys Leu Glu Arg Arg
        115                 120                 125

Leu Tyr Arg Val Ser Leu Ala Cys Val Cys Val Arg Pro Arg Val Met
    130                 135                 140

Gly Gly Gly Gly Gly Ser Gly Gly Gly Gly Ser Gly Gly Gly Gly Ser
145                 150                 155                 160

Asp Ala His Lys Ser Glu Val Ala His Arg Phe Lys Asp Leu Gly Glu
                165                 170                 175

Glu Asn Phe Lys Ala Leu Val Leu Ile Ala Phe Ala Gln Tyr Leu Gln
            180                 185                 190

Gln Cys Pro Phe Glu Asp His Val Lys Leu Val Asn Glu Val Thr Glu
        195                 200                 205

Phe Ala Lys Thr Cys Val Ala Asp Glu Ser Ala Glu Asn Cys Asp Lys
    210                 215                 220

Ser Leu His Thr Leu Phe Gly Asp Lys Leu Cys Thr Val Ala Thr Leu
225                 230                 235                 240

Arg Glu Thr Tyr Gly Glu Met Ala Asp Cys Cys Ala Lys Gln Glu Pro
                245                 250                 255

Glu Arg Asn Glu Cys Phe Leu Gln His Lys Asp Asp Asn Pro Asn Leu
            260                 265                 270

Pro Arg Leu Val Arg Pro Glu Val Asp Val Met Cys Thr Ala Phe His
        275                 280                 285

Asp Asn Glu Glu Thr Phe Leu Lys Lys Tyr Leu Tyr Glu Ile Ala Arg
    290                 295                 300

Arg His Pro Tyr Phe Tyr Ala Pro Glu Leu Leu Phe Phe Ala Lys Arg
305                 310                 315                 320

Tyr Lys Ala Ala Phe Thr Glu Cys Cys Gln Ala Ala Asp Lys Ala Ala
                325                 330                 335

Cys Leu Leu Pro Lys Leu Asp Glu Leu Arg Asp Glu Gly Lys Ala Ser
            340                 345                 350

Ser Ala Lys Gln Arg Leu Lys Cys Ala Ser Leu Gln Lys Phe Gly Glu
        355                 360                 365

Arg Ala Phe Lys Ala Trp Ala Val Ala Arg Leu Ser Gln Arg Phe Pro
    370                 375                 380

Lys Ala Glu Phe Ala Glu Val Ser Lys Leu Val Thr Asp Leu Thr Lys
385                 390                 395                 400

Val His Thr Glu Cys Cys His Gly Asp Leu Leu Glu Cys Ala Asp Asp
                405                 410                 415

Arg Ala Asp Leu Ala Lys Tyr Ile Cys Glu Asn Gln Asp Ser Ile Ser
            420                 425                 430

Ser Lys Leu Lys Glu Cys Cys Glu Lys Pro Leu Leu Glu Lys Ser His
        435                 440                 445

Cys Ile Ala Glu Val Glu Asn Asp Glu Met Pro Ala Asp Leu Pro Ser
    450                 455                 460

Leu Ala Ala Asp Phe Val Glu Ser Lys Asp Val Cys Lys Asn Tyr Ala
465                 470                 475                 480

Glu Ala Lys Asp Val Phe Leu Gly Met Phe Leu Tyr Glu Tyr Ala Arg
                485                 490                 495

Arg His Pro Asp Tyr Ser Val Val Leu Leu Leu Arg Leu Ala Lys Thr
            500                 505                 510

Tyr Glu Thr Thr Leu Glu Lys Cys Cys Ala Ala Ala Asp Pro His Glu
        515                 520                 525

Cys Tyr Ala Lys Val Phe Asp Glu Phe Lys Pro Leu Val Glu Glu Pro
    530                 535                 540

Gln Asn Leu Ile Lys Gln Asn Cys Glu Leu Phe Glu Gln Leu Gly Glu
545                 550                 555                 560

Tyr Lys Phe Gln Asn Ala Leu Leu Val Arg Tyr Thr Lys Lys Val Pro
                565                 570                 575

Gln Val Ser Thr Pro Thr Leu Val Glu Val Ser Arg Asn Leu Gly Lys
            580                 585                 590

Val Gly Ser Lys Cys Cys Lys His Pro Glu Ala Lys Arg Met Pro Cys
        595                 600                 605

Ala Glu Asp Tyr Leu Ser Val Val Leu Asn Gln Leu Cys Val Leu His
    610                 615                 620

Glu Lys Thr Pro Val Ser Asp Arg Val Thr Lys Cys Cys Thr Glu Ser
625                 630                 635                 640

Leu Val Asn Arg Arg Pro Cys Phe Ser Ala Leu Glu Val Asp Glu Thr

                645                 650                 655

Tyr Val Pro Lys Glu Phe Asn Ala Glu Thr Phe Thr Phe His Ala Asp
            660                 665                 670

Ile Cys Thr Leu Ser Glu Lys Glu Arg Gln Ile Lys Lys Gln Thr Ala
        675                 680                 685

Leu Val Glu Leu Val Lys His Lys Pro Lys Ala Thr Lys Glu Gln Leu
    690                 695                 700

Lys Ala Val Met Asp Asp Phe Ala Ala Phe Val Glu Lys Cys Cys Lys
705                 710                 715                 720

Ala Asp Asp Lys Glu Thr Cys Phe Ala Glu Glu Gly Lys Lys Leu Val
                725                 730                 735

Ala Ala Ser Gln Ala Ala Leu Gly Leu
            740                 745

<210> 19
<211> 396
<212> PRT
<213> Artificial sequence

<400> 19

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro

115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Gly Gly Gly Gly Ser Gly Gly Gly Gly Ser Gly
225                 230                 235                 240

Gly Gly Gly Ser Asp Cys Asp Ile Glu Gly Lys Asp Gly Lys Gln Tyr
                245                 250                 255

Glu Ser Val Leu Met Val Ser Ile Asp Gln Leu Leu Asp Ser Met Lys
            260                 265                 270

Glu Ile Gly Ser Asn Cys Leu Asn Asn Glu Phe Asn Phe Phe Lys Arg
        275                 280                 285

His Ile Cys Asp Ala Asn Lys Glu Gly Met Phe Leu Phe Arg Ala Ala
    290                 295                 300

Arg Lys Leu Arg Gln Phe Leu Lys Met Asn Ser Thr Gly Asp Phe Asp
305                 310                 315                 320

Leu His Leu Leu Lys Val Ser Glu Gly Thr Thr Ile Leu Leu Asn Cys
                325                 330                 335

Thr Gly Gln Val Lys Gly Arg Lys Pro Ala Ala Leu Gly Glu Ala Gln
            340                 345                 350

Pro Thr Lys Ser Leu Glu Glu Asn Lys Ser Leu Lys Glu Gln Lys Lys
        355                 360                 365

Leu Asn Asp Leu Cys Phe Leu Lys Arg Leu Leu Gln Glu Ile Lys Thr
    370                 375                 380

Cys Trp Asn Lys Ile Leu Met Gly Thr Lys Glu His
385                 390                 395

<210>  20
<211>  389
<212>  PRT
<213>  Artificial sequence

<400>  20

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Gly Gly Gly Gly Ser Gly Gly Gly Gly Ser Gly
225                 230                 235                 240

Gly Gly Gly Ser Tyr Ser His Trp Pro Ser Cys Cys Pro Ser Lys Gly
                245                 250                 255

Gln Asp Thr Ser Glu Glu Leu Leu Arg Trp Ser Thr Val Pro Val Pro
            260                 265                 270

Pro Leu Glu Pro Ala Arg Pro Asn Arg His Pro Glu Ser Cys Arg Ala
        275                 280                 285

Ser Glu Asp Gly Pro Leu Asn Ser Arg Ala Ile Ser Pro Trp Arg Tyr
    290                 295                 300

Glu Leu Asp Arg Asp Leu Asn Arg Leu Pro Gln Asp Leu Tyr His Ala
305                 310                 315                 320

Arg Cys Leu Cys Pro His Cys Val Ser Leu Gln Thr Gly Ser His Met
                325                 330                 335

Asp Pro Arg Gly Asn Ser Glu Leu Leu Tyr His Asn Gln Thr Val Phe
            340                 345                 350

Tyr Arg Arg Pro Cys His Gly Glu Lys Gly Thr His Lys Gly Tyr Cys
        355                 360                 365

Leu Glu Arg Arg Leu Tyr Arg Val Ser Leu Ala Cys Val Cys Val Arg
    370                 375                 380

Pro Arg Val Met Gly
385

<210>  21
<211>  8
<212>  PRT
<213>  Artificial sequence

<220>
<223>  uPA sensitive cleavage sequence

<400>  21

Leu Ser Gly Arg Ser Asp Asn His

1               5


<210>  22
<211>  4
<212>  PRT
<213>  Artificial sequence

<220>
<223>  cysteine protease sensitive cleavage sequence

<400>  22

Ala Ala Asn Leu
1


<210>  23
<211>  4
<212>  PRT
<213>  Artificial sequence

<220>
<223>  cysteine protease sensitive cleavage sequence

<400>  23

Ala Ala Asn Val
1


<210>  24
<211>  6
<212>  PRT
<213>  Artificial sequence

<220>
<223>  MMP-1 sensitive cleavage sequence

<400>  24

Pro Leu Gly Leu Trp Ala
1               5


<210>  25
<211>  7
<212>  PRT
<213>  Artificial sequence

<220>
<223>  MMP-2/9 sensitive cleavage sequence

<400>  25

Pro Ala Ala Leu Val Gly Ala
1               5


<210>  26
<211>  10
<212>  PRT
<213>  Artificial sequence

<220>
<223>  MMP-14 sensitive cleavage sequence

<400>  26

Ser Gly Arg Ile Gly Phe Leu Arg Thr Ala
1               5                   10


<210>  27
<211>  4
<212>  PRT
<213>  Artificial sequence

<220>
<223>  Cathepsin B sensitive cleavage sequence

<400>  27

Gly Phe Leu Gly
1


<210>  28
<211>  5
<212>  PRT
<213>  Artificial sequence

<220>
<223>  flexible linker

<400>  28

Gly Gly Gly Gly Ser
1               5

<210>  29
<211>  5
<212>  PRT
<213>  Artificial sequence

<220>
<223>  ¦Á-helix linker

<400>  29

Glu Ala Ala Ala Lys
1               5


<210>  30
<211>  338
<212>  PRT
<213>  Artificial sequence

<220>
<223>  IL-25-IL7 recombinant protein

<400>  30

Tyr Ser His Trp Pro Ser Cys Cys Pro Ser Lys Gly Gln Asp Thr Ser
1               5                   10                  15

Glu Glu Leu Leu Arg Trp Ser Thr Val Pro Val Pro Pro Leu Glu Pro
            20                  25                  30

Ala Arg Pro Asn Arg His Pro Glu Ser Cys Arg Ala Ser Glu Asp Gly
        35                  40                  45

Pro Leu Asn Ser Arg Ala Ile Ser Pro Trp Arg Tyr Glu Leu Asp Arg
    50                  55                  60

Asp Leu Asn Arg Leu Pro Gln Asp Leu Tyr His Ala Arg Cys Leu Cys
65                  70                  75                  80

Pro His Cys Val Ser Leu Gln Thr Gly Ser His Met Asp Pro Arg Gly
                85                  90                  95

Asn Ser Glu Leu Leu Tyr His Asn Gln Thr Val Phe Tyr Arg Arg Pro
            100                 105                 110

Cys His Gly Glu Lys Gly Thr His Lys Gly Tyr Cys Leu Glu Arg Arg
        115                 120                 125

Leu Tyr Arg Val Ser Leu Ala Cys Val Cys Val Arg Pro Arg Val Met
    130                 135                 140

Gly Gly Gly Ser Gly Leu Ser Gly Arg Ser Asp Asn His Gly Gly Gly
145                 150                 155                 160

Gly Ser Asp Cys Asp Ile Glu Gly Lys Asp Gly Lys Gln Tyr Glu Ser
                165                 170                 175

Val Leu Met Val Ser Ile Asp Gln Leu Leu Asp Ser Met Lys Glu Ile
            180                 185                 190

Gly Ser Asn Cys Leu Asn Asn Glu Phe Asn Phe Phe Lys Arg His Ile
        195                 200                 205

Cys Asp Ala Asn Lys Glu Gly Met Phe Leu Phe Arg Ala Ala Arg Lys
    210                 215                 220

Leu Arg Gln Phe Leu Lys Met Asn Ser Thr Gly Asp Phe Asp Leu His
225                 230                 235                 240

Leu Leu Lys Val Ser Glu Gly Thr Thr Ile Leu Leu Asn Cys Thr Gly
                245                 250                 255

Gln Val Lys Gly Arg Lys Pro Ala Ala Leu Gly Glu Ala Gln Pro Thr

260                 265                 270

Lys Ser Leu Glu Glu Asn Lys Ser Leu Lys Glu Gln Lys Lys Leu Asn
        275                 280                 285

Asp Leu Cys Phe Leu Lys Arg Leu Leu Gln Glu Ile Lys Thr Cys Trp
    290                 295                 300

Asn Lys Ile Leu Met Gly Thr Lys Glu His Gly Gly Gly Gly Ser Gly
305                 310                 315                 320

Gly Gly Gly Ser Gly Gly Gly Gly Ser Cys Arg Gly Asp Lys Gly Pro
                325                 330                 335

Asp Cys

<210>  31
<211>  489
<212>  PRT
<213>  Artificial sequence

<220>
<223>  IL-25-IL-7-IL-2 recombinant protein

<400>  31

Tyr Ser His Trp Pro Ser Cys Cys Pro Ser Lys Gly Gln Asp Thr Ser
1               5                   10                  15

Glu Glu Leu Leu Arg Trp Ser Thr Val Pro Val Pro Pro Leu Glu Pro
            20                  25                  30

Ala Arg Pro Asn Arg His Pro Glu Ser Cys Arg Ala Ser Glu Asp Gly
        35                  40                  45

Pro Leu Asn Ser Arg Ala Ile Ser Pro Trp Arg Tyr Glu Leu Asp Arg
    50                  55                  60

Asp Leu Asn Arg Leu Pro Gln Asp Leu Tyr His Ala Arg Cys Leu Cys
65                  70                  75                  80

Pro His Cys Val Ser Leu Gln Thr Gly Ser His Met Asp Pro Arg Gly
                85                  90                  95

Asn Ser Glu Leu Leu Tyr His Asn Gln Thr Val Phe Tyr Arg Arg Pro
            100                 105                 110

Cys His Gly Glu Lys Gly Thr His Lys Gly Tyr Cys Leu Glu Arg Arg
        115                 120                 125

Leu Tyr Arg Val Ser Leu Ala Cys Val Cys Val Arg Pro Arg Val Met
    130                 135                 140

Gly Gly Gly Ser Gly Leu Ser Gly Arg Ser Asp Asn His Gly Gly Gly
145                 150                 155                 160

Gly Ser Asp Cys Asp Ile Glu Gly Lys Asp Gly Lys Gln Tyr Glu Ser
                165                 170                 175

Val Leu Met Val Ser Ile Asp Gln Leu Leu Asp Ser Met Lys Glu Ile
            180                 185                 190

Gly Ser Asn Cys Leu Asn Asn Glu Phe Asn Phe Phe Lys Arg His Ile
        195                 200                 205

Cys Asp Ala Asn Lys Glu Gly Met Phe Leu Phe Arg Ala Ala Arg Lys
    210                 215                 220

Leu Arg Gln Phe Leu Lys Met Asn Ser Thr Gly Asp Phe Asp Leu His
225                 230                 235                 240

Leu Leu Lys Val Ser Glu Gly Thr Thr Ile Leu Leu Asn Cys Thr Gly
                245                 250                 255

Gln Val Lys Gly Arg Lys Pro Ala Ala Leu Gly Glu Ala Gln Pro Thr
            260                 265                 270

Lys Ser Leu Glu Glu Asn Lys Ser Leu Lys Glu Gln Lys Lys Leu Asn
        275                 280                 285

Asp Leu Cys Phe Leu Lys Arg Leu Leu Gln Glu Ile Lys Thr Cys Trp
    290                 295                 300

Asn Lys Ile Leu Met Gly Thr Lys Glu His Gly Gly Gly Ser Gly Leu
305                 310                 315                 320

Ser Gly Arg Ser Asp Asn His Gly Gly Gly Gly Ser Ala Pro Thr Ser
                325                 330                 335

Ser Ser Thr Lys Lys Thr Gln Leu Gln Leu Glu His Leu Leu Leu Asp
            340                 345                 350

Leu Gln Met Ile Leu Asn Gly Ile Asn Asn Tyr Lys Asn Pro Lys Leu
        355                 360                 365

Thr Ala Met Leu Thr Lys Lys Phe Tyr Met Pro Lys Lys Ala Thr Glu

370                 375                 380

Leu Lys His Leu Gln Cys Leu Glu Glu Glu Leu Lys Pro Leu Glu Glu
385                 390                 395                 400

Val Leu Asn Leu Ala Gln Ser Lys Asn Phe His Leu Arg Pro Arg Asp
                405                 410                 415

Leu Ile Ser Asn Ile Asn Val Ile Val Leu Glu Leu Lys Gly Ser Glu
            420                 425                 430

Thr Thr Phe Met Cys Glu Tyr Ala Asp Glu Thr Ala Thr Ile Val Glu
        435                 440                 445

Phe Leu Asn Arg Trp Ile Thr Phe Cys Gln Ser Ile Ile Ser Thr Leu
    450                 455                 460

Thr Gly Gly Gly Gly Ser Gly Gly Gly Gly Ser Gly Gly Gly Gly Ser
465                 470                 475                 480

Cys Arg Gly Asp Lys Gly Pro Asp Cys
                485

<210>  32
<211>  6
<212>  PRT
<213>  Artificial sequence

<220>
<223>  MMP-14 sensitive cleavage sequence

<400>  32

Pro Ala Gly Leu Val Gly
1               5

## Description

### FIELD

The present application relates to interleukin combinations or recombinant proteins for the prevention and/or treatment of malignant tumors, various products formed therefrom, and use thereof.

### BACKGROUND

γc-cytokine, also known as γc-family cytokine or common cytokine receptor gamma-chain family, consists of six members, namely IL-2, IL-4, IL-7, IL-9, IL-15 and IL-21. All members of this family conduct signal through a receptor complex containing a common cytokine receptor gamma chain subunit. The receptors for IL-4, IL-7, IL-9, and IL-21 are heterodimeric complexes composed of a common gamma chain and a unique cytokine-specific subunit; the receptors for IL-2 and IL-15 are heterotrimeric complexes composed of IL-2Rα/IL-15Rα, IL-2/IL-15 Rβ, and a common γ-chain.

Signaling pathways for activation of the common cytokine receptor γ-chain family include the PI3-K-AKT signaling pathway, the Ras-MAPK signaling pathway, and the JAK-STAT signaling pathway. Cytokines of this family are critical for the establishment and maintenance of normal immune system functions and have unique and overlapping effects on many types of cells including T cells, B cells, natural killer cells, mast cells, and myeloid and erythroid progenitor cells.

IL-2 was originally identified as a T cell growth factor and has been shown to play a crucial role in maintaining T cell homeostasis and preventing autoreactivity. IL-4 is necessary for Th2 and Th9 cell differentiation, regulation of immunoglobulin class switching, and promotion of mast cell survival and proliferation. IL-7 is necessary for T cell development and homeostasis proliferation, mouse B cell development, and memory T cell production. IL-9 promotes the growth of mouse T cells and mast cells, regulates the production of immunoglobulin by B cells, enhances the expression of proteases by mast cells, promotes goblet cell proliferation and mucus secretion. IL-15 is necessary for NK cell development, survival and activation, NKT cell and intraepithelial lymphocyte homeostasis, and maintenance of primary and memory CD8⁺ T cells. The recently reported family member IL-21 is necessary for Th17 cell differentiation and follicular helper T cell production. In addition, it also regulates B cell activity and cytotoxicity of CD8 ⁺ T cells and NK cells.

IL-25, a pro-inflammatory cytokine, is a member of the IL-17 family, which is highly expressed in certain organs such as testis, prostate, and spleen, and is lowly expressed in other organs including normal mammary glands. Although IL-25 is structurally related to the IL-17 family, it is distinct from IL-17 and other family members in biological functions. IL-25 is highly expressed in activated Th2 cells and non-T cells, has a Th2 cytokine-promoting response, has a protective immune response against helminth infection, and negatively regulates Th17 function in autoimmune inflammation.

IL-33 is a new member of the IL-1 family discovered in 2005. It activates mast cells, lymphocytes and eosinophils to produce class Th2 cytokines, and plays an important role in inflammation, infection, and autoimmune diseases.

IL-25 and IL-33 are important for the body's anti-helminth immunity, anti-fungal infections, allergic inflammation and mucosal function. Certain stimuli, such as fungal infections, can trigger the release of IL25 and IL33 from epithelial cells, both of which activate type 2 Innate lymphoid cells (ILC2) and cause a population of ILC2 to expand and to release IL5 and IL13, which in turn promote amplification and activation of eosinophils, although ILC2s activated by IL-25 and IL-33 are different. Activated eosinophils can produce cytotoxic effects on target cells. The ILC2 cell population itself has strong plasticity. For example, IL13⁺ILC2 in peripheral blood can obtain the ability to produce IFNγ, which may be related to autoimmune disease reaction such as colitis.

### SUMMARY

In a first aspect, the present disclosure provides a pharmaceutical combination for preventing and/or treating a malignant tumor comprising:
i) at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active part or variant thereof; and
ii) at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

In one embodiment, at least one of γc-cytokine in the pharmaceutical combination provided in the present disclosure comprises IL-2, IL-4, IL-7, IL-9, IL-15 or IL-21. In another embodiment, at least one of γc-cytokine in the pharmaceutical combination provided in the present disclosure comprises IL-2, IL-7, IL-9 or IL-15. In still another embodiment, at least one of γc-cytokine in the pharmaceutical combination provided in the present disclosure comprises IL-7 or IL-2.

Thus, in certain embodiments, the pharmaceutical combination provided in the present disclosure includes or consists of:
i) at least one of IL-2, IL-4, IL-7, IL-9, IL-15 or IL-21 or an active part or variant thereof, or a vector or a cell or a population of cells capable of producing at least one of IL-2, IL-4, IL-7, IL-9, IL-15 or IL-21 or an active part or variant thereof, or a nucleic acid molecule capable of encoding at least one of IL-2, IL-4, IL-7, IL- 9, IL-15 or IL-21 or an active part or variant thereof; and
ii) at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

In a second aspect, the present disclosure provids a kit or a pharmaceutical composition for preventing and/or treating a malignant tumor comprising:
a first component: at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active part or variant thereof; and
a second component: at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

In a particular embodiment, the first component and the second component in the kit provided in the present disclosure are present in separate pharmaceutical compositions; or present in the same pharmaceutical composition.

In a third aspect, the present disclosure provides a recombinant protein for preventing and/or treating a malignant tumor comprising:
at least one of γc-cytokine or an active part or variant thereof; and
at least one of IL-25 or IL-33 or an active part or variant thereof.

In one embodiment, at least one of yc-cytokine in the recombinant protein provided in the present disclosure comprises IL-2, IL-4, IL-7, IL-9, IL-15 or IL-21. In another embodiment, at least one of γc-cytokine in the recombinant protein provided in the present disclosure comprises IL-2, IL-7, IL-9 or IL-15. In still another embodiment, at least one of γc-cytokine in the recombinant protein provided in the present disclosure comprises IL-7 or IL-2.

In one embodiment, the recombinant protein provided in the present disclosure comprises:
an amino acid sequence as set forth in any one of SEQ ID NOs: 1-6 or an active part or variant thereof, or an amino acid sequence having at least 70%, 80%, 90%, 95% or 99% identity with an amino acid sequence as set forth in any one of SEQ ID NOs: 1-6 or an active part or variant thereof; and
an amino acid sequence as set forth in SEQ ID NO: 7 or 8 or an active part or variant thereof, or an amino acid sequence having at least 70%, 80%, 90%, 95% or 99% identity with the amino acid sequence as set forth in SEQ ID NO: 7 or 8 or an active part or variant thereof .

In one embodiment, the recombinant protein provided in the present disclosure comprises one or more linkers, preferably peptide linkers that are cleavable *in vivo* or intratumorally.

In one embodiment, the linker is selected from the group consisting of one or more of: uPA linker (urokinase-type plasminogen activator substrate sequence, SEQ ID NO: 21), legumain linker (cysteine protease, SEQ ID NOs: 22 and 23), MMP-1 linker

(SEQ ID NO: 24), MMP-2/9 linker (SEQ ID NO: 25), MMP-14 linker (SEQ ID NO: 26 and 32), substrate peptide linker of cathepsin B (SEQ ID NO: 27), glycine-serine linker (GGGGS)ₙ (n = 1-3) (SEQ ID NO: 28), α-helix forming peptide linker (EAAAK)ₙ (n=2-5) (SEQ ID NO: 29), *in vivo* cleavable disulfide bond linker.

In one embodiment, the recombinant protein provided in the present disclosure comprises the sequence as set forth in SEQ ID NO: 30 or 31.

In a fourth aspect, the present disclosure provides a recombinant nucleic acid molecule for preventing and/or treating a malignant tumor comprising:
a first nucleotide sequence selected from the group consisting of: a nucleotide sequence as set forth in any one of SEQ ID NOs: 9-14 or a variant thereof, or a nucleotide sequence having at least 70%, 80%, 90%, 95% or 99% identity with the nucleotide sequence as set forth in any one of SEQ ID NOs: 9-14 or variant thereof ; and/or
a second nucleotide sequence selected from the group consisting of: a nucleotide sequence as set forth in SEQ ID NOs: 15 or 16 or a variant thereof, or a nucleotide sequence having at least 70%, 80%, 90%, 95% or 99% identity with the nucleotide sequence as set forth in SEQ ID NOs: 15 or 16 or a variant thereof.

In a fifth aspect, the present disclosure provides a vector for preventing and/or treating a malignant tumor, comprising:
a first nucleotide sequence selected from the group consisting of: a nucleotide sequence as set forth in any one of SEQ ID NOs: 9-14 or a variant thereof, or a nucleotide sequence having at least 70%, 80%, 90%, 95% or 99% identity with the nucleotide sequence as set forth in any one of SEQ ID NOs: 9-14 or variant thereof ; and/or
a second nucleotide sequence selected from the group consisting of: a nucleotide sequence as set forth in SEQ ID NO: 15 or 16 or a variant thereof, or a nucleotide sequence having at least 70%, 80%, 90%, 95% or 99% identity with the nucleotide sequence as set forth in SEQ ID NO: 15 or 16 or a variant thereof.

In one embodiment, the vector provided in the present disclosure is selected from a plasmid or viral vector, including but not limited to: an adenoviral vector, an adeno-associated viral vector, a retroviral vector, a lentiviral vector, a sleeping beauty or a PiggyBac transposon system, a CRISPR/Cas9 knock-in system or any other suitable vectors.

In another embodiment, the first nucleotide sequence and the second nucleotide sequence provided in the present disclosure are present in separate vectors; or wherein the first nucleotide sequence and the second nucleotide sequence are present in the same vector.

In a sixth aspect, the present disclosure provides a cell or a population of cells for preventing and/or treating a malignant tumor, which is capable of producing the recombinant protein provided in the present disclosure, or comprises the recombinant nucleic acid molecule or vector described in the present disclosure.

In one embodiment, the cell or population of cells provided in the present disclosure is selected from the group consisting of an autologous cell, an allogeneic cell, including but not limited to dendritic cells, T cells, macrophages, eosinophils; transformed homologous or xenogeneic cells, including but not limited to Chinese hamster ovary cells (CHO), baby hamster kidney cells (BHK), human embryonic kidney HEK293 derived cells or any other suitable cells.

In another embodiment, the cell or population of cells provided in the present disclosure can be microencapsulated, for example, the transformed homologous or xenogeneic cells provided in the present disclosure can be microencapsulated. Alternatively, the cell or population of cells provided in the present disclosure can be microencapsulated with a hydrogel such as sodium alginate.

In still another embodiment, the recombinant protein provided in the present disclosure is produced by expression of different cells or populations of cells, or by expression of the same cell or population of cells; or the recombinant nucleic acid molecule or vector provided in the present disclosure is comprised in different cells or populations of cells, or comprised in the same cell or population of cells.

In a seventh aspect, the present disclosure provides a method for preventing and/or treating a malignant tumor comprising administering to individuals in need thereof a prophylactically and/or therapeutically effective amount of the pharmaceutical combination provided in the present disclosure, or the kit provided in the present disclosure, or the recombinant protein provided in the present disclosure, or the recombinant nucleic acid molecule provided in the present disclosure, or the vector provided in the present disclosure, or the cell or population of cells provided in the present disclosure.

In one embodiment, when the recombinant protein provided in the present disclosure is administered to individuals in need thereof, the dose administered is 0.0001 to 10 mg/kg body weight per time, once every 0-30 days. In another embodiment, the required corresponding dose infused by an automated timed quantitative subcutaneous continuous infusion pump, such as an insulin pump, can be selected.

In another embodiment, when the vector provided in the present disclosure, such as a virus, is intratumorally administered to individuals in need thereof, the dose administered is 10⁸-10¹³ pfu virus/tumor/time; once every 0-30 days.

In yet another embodiment, when the cell or population of cells provided in the present disclosure is administered to individuals in need thereof, the dose administered is 10⁵-10¹¹ cells/kg body weight/time, once every 0-100 days.

In an eighth aspect, the present disclosure provides the use of the following combination in the prevention and/or treatment of a malignant tumor, or in the manufacture of a medicament for the prevention and/or treatment of a malignant tumor:
i) at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active part or variant thereof; and
ii) at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

In the above aspects, at least one of γc-cytokine provided in the present disclosure comprises IL-2, IL-4, IL-7, IL-9, IL-15 or IL-21; preferably IL-2, IL-7, IL-9 or IL-15; more preferably IL-7 or IL-2.

In the above aspects, at least one of γc-cytokine provided in the present disclosure, i.e., IL-2, IL-4, IL-7, IL-9, IL-15 or IL-21, or an active part or variant thereof, and/or the at least one of IL-25 or IL-33 or an active part or variant thereof, and/or the recombinant protein provided in the present disclosure further comprises one or more modifications selected from the group consisting of: fusion with human immunoglobulin Fc fragment or a variant thereof; fusion with human serum albumin or a variant thereof; fusion with a tumor-penetrating peptide; fusion with a tumor-targeting single-chain or single-domain antibody; PEGylation; HESylation; Xtenylation; PASylation; or any other suitable chemical modifications.

In the above aspects, the involved malignant tumor is selected from the group consisting of: fibrosarcoma, mucinous sarcoma, liposarcoma, chondrosarcoma, osteosarcoma, chordoma, angiosarcoma, endothelial sarcoma, lymphangiosarcoma, lymphangial endothelial sarcoma, synovial sarcoma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous gland cancer, papillary cancer, papillary adenocarcinoma, carcinoma, bronchial carcinoma, medullary carcinoma, renal cell carcinoma, liver cancer, cholangiocarcinoma, choriocarcinoma, seminoma, embryonal carcinoma, nephroblastoma, cervical cancer, testicular tumor, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, meningioma, oligodendroglioma, melanoma, neuroblastoma, and retinoblastoma.

### BRIEF DESCRIPTION OF THE DRAWINGS

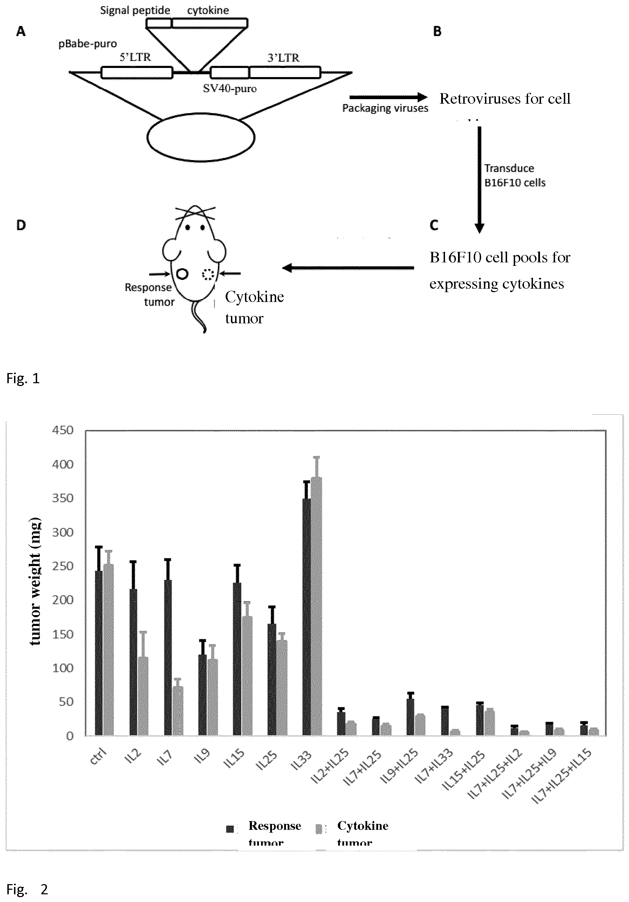

Fig. 1 shows a flow chart of drug screening for B16F10 melanoma cytokine combination therapy. A is a retroviral vector constructed with mouse cytokines; B shows that HEK293T cells co-transfected with retroviral vector and packaging helper plasmid pCL-Ampho produce retroviral particles capable of expressing cytokines; C shows generation and enrichment of cell pools for cytokine production. B16F10 cells transduced with corresponding retroviruses were enriched by screening with puromycin; D relates to verifying the effects of cytokines and combinations thereof on tumor growth. The primary B16F10 cells were injected into the left side of mice as response tumors, and a cell pool for producing single cytokine or two cell pools for producing different cytokines were injected into the right side as cytokine tumors.
Fig. 2 exemplarily shows the results of the inhibitory effects of some cytokines on tumor growth (n=4 per group). Primary B16F10 cells or B16F10 transduced with empty retroviral vector are used in the control group. The tumor was removed and weighed 16 days after inoculating cells.

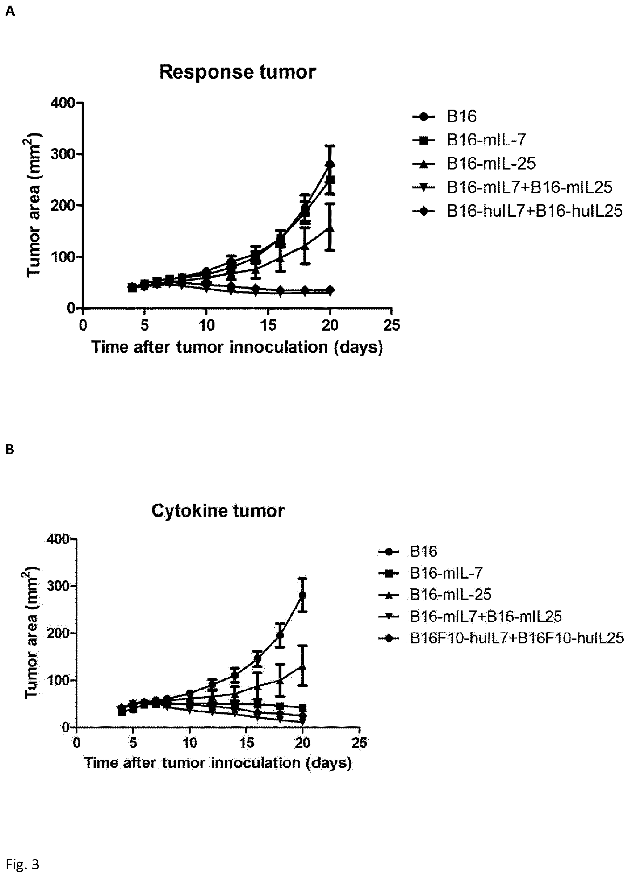

Fig. 3 shows that tumor growth dynamics, suggesting a synergistic anti-tumor effect of the combination of IL-7 and IL-25 in C57BL/6 mice. 5×10⁵ B16F10 primary cells were injected intradermally (i.d.) into the left side of C57BL/6 mice (as response tumors), whereas a cell pool for producing IL-7 or IL-25 or a mixed cell pool for producing the two cytokines (1 million each) was injected intradermally into the right side (as a drug tumor). The area of the response tumor (A) and the area of the cytokine-producing tumor (B) (n = 10 per group) were measured 4 days after tumor cell injection.

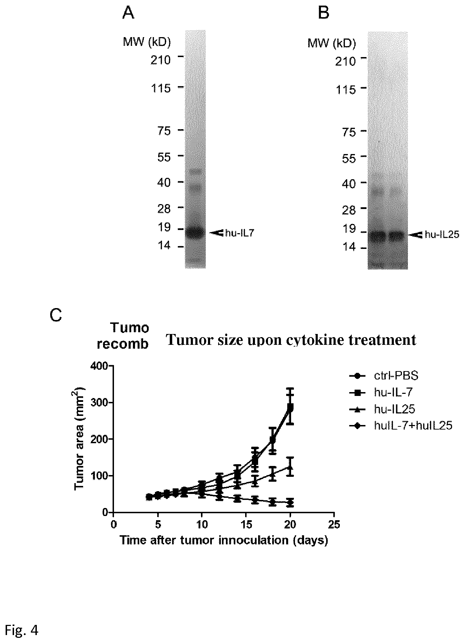

Fig. 4 shows the Coomassie brilliant blue staining map of the recombinant protein with 6xHis tag expressed in *E. coli* BL21 (DE3) (10 µg protein per lane), where A is IL-7, B is IL-25, and C is the therapeutic response dynamics of tumors to hu-IL-7, hu-IL-25 or the combination of huIL-7+huIL-25. 5×10⁵ B16F10 primary cells were injected intradermally on both sides of C57BL/6 mice (10 in each group). After 24 hours, the mice were injected intraperitoneally twice daily with IL-7, IL-25, IL-7+ IL-25 dissolved in PBS (5 µg each/time). The tumor area was measured daily 5 days after tumor cell injection.

### DESCRIPTION OF SEQUENCES

Unless otherwise indicated, the amino acid sequences of the signal peptides or corresponding nucleotide sequences thereof are shown in bold in the following sequences.
SEQ ID NO: 1 shows the amino acid sequence of human IL-2. 
MYRMQLLSCIALSLALVTNSAPTSSSTKKTQLQLEHLLLDLQMILNGINNYK
NPKLTRMLTFKFYMPKKATELKHLQCLEEELKPLEEVLNLAQSKNFHLRPRDL
ISNINVIVLELKGSETTFMCEYADETATIVEFLNRWITFCQSIISTLT
SEQ ID NO: 2 shows the amino acid sequence of human IL-4.
SEQ ID NO: 3 shows the amino acid sequence of human IL-7, in which the underlined amino acid residues in italics (positions 96-114) can be deleted from hu-IL-7 sequence and the resultant sequence is a splice variant. 
MFHVSFRYIF GLPPLILVLL PVASSDCDIE GKDGKQYESV LMVSIDQLLD
SMKEIGSNCL NNEFNFFKRH ICDANKEGMF LFRAARKLRQ FLKMNSTGDF
DLHLLKVSEG TTILLNCTGQ VKGRKPAALG EAQPTKSLEE NKSLKEQKKL
NDLCFLKRLL QEIKTCWNKI LMGTKEH
SEQ ID NO: 4 shows the amino acid sequence of human IL-9. 
MLLAMVLTSALLLCSVAGQGCPTLAGILDINFLINKMQEDPASKCHCSANVT
SCLCLGIPSDNCTRPCFSERLSQMTNTTMQTRYPLIFSRVKKSVEVLKNNKCPY
FSCEQPCNQTTAGNALTFLKSLLEIFQKEKMRGMRGKI
SEQ ID NO: 5 shows the amino acid sequence of human IL-15. 
MRISKPHLRSISIQCYLCLLLNSHFLTEAGIHVFILGCFSAGLPKTEANWVNV
ISDLKKIEDLIQSMHIDATLYTESDVHPSCKVTAMKCFLLELQVISLESGDASIH
DTVENLIILANNSLSSNGNVTESGCKECEELEEKNIKEFLQSFVHIVQMFINTS
SEQ ID NO: 6 shows the amino acid sequence of human IL-21. 
MRSSPGNMERIVICLMVIFLGTLVHKSSSQGQDRHMIRMRQLIDIVDQLKN
YVNDLVPEFLPAPEDVETNCEWSAFSCFQKAQLKSANTGNNERIINVSIKKLK
RKPPSTNAGRRQKHRLTCPSCDSYEKKPPKEFLERFKSLLQKMIHQHLSSRTH
GSEDS
SEQ ID NO: 7 shows the amino acid sequence of human IL-25, in which the signal peptide sequence in bold italics in a splice variant hu-IL-25 is different and can be replaced with the amino acid Y, but the mature IL-25 sequence is identical. 
MRERPRLGEDSSLISLFLQVVAFLAMVMGTHTYSHWPSCCPSKGQDTSEEL
LRWSTVPVPPLEPARPNRHPESCRASEDGPLNSRAISPWRYELDRDLNRLPQD
LYHARCLCPHCVSLQTGSHMDPRGNSELLYHNQTVFYRRPCHGEKGTHKGY
CLERRLYRVSLACVCVRPRVMG
SEQ ID NO: 8 shows the amino acid sequence of human IL-33, in which shown in bold is the amino acid sequence comprised in the propeptide. 
MKPKMKYSTNKISTAKWKNTASKALCFKLGKSQQKAKEVCPMYFMKL
RSGLMIKKEACYFRRETTKRPSLKTGRKHKRHLVLAACQQQSTVECFAF
GISGVQKYTRALHDSSITGISPITEYLASLSTYNDQSITFALEDESYEIYVEDLK
KDEKKDKVLLSYYESQHPSNESGDGVDGKMLMVTLSPTKDFWLHANNKEH
SVELHKCEKPLPDQAFFVLHNMHSNCVSFECKTDPGVFIGVKDNHLALIKVD
SSENLCTENILFKLSET
SEQ ID NO: 9 shows the nucleotide sequence of human IL-2. 
atgtacaggatgcaactcctgtcttgcattgcactaagtcttgcacttgtcacaaacagtgcacctacttcaagttctaca
aagaaaacacagctacaactggagcatttactgctggatttacagatgattttgaatggaattaataattacaagaatcccaaa
ctcaccaggatgctcacatttaagttttacatgcccaagaaggccacagaactgaaacatcttcagtgtctagaagaagaact
caaacctctggaggaagtgctaaatttagctcaaagcaaaaactttcacttaagacccagggacttaatcagcaatatcaac
gtaatagttctggaactaaagggatctgaaacaacattcatgtgtgaatatgctgatgagacagcaaccattgtagaatttctg
 aacagatggattaccttttgtcaaagcatcatctcaacactgacttga
SEQ ID NO: 10 shows the nucleotide sequence of human IL-4. 
atgggtctcacctcccaactgcttccccctctgttcttcctgctagcatgtgccggcaactttgtccacggacacaagtg
cgatatcaccttacaggagatcatcaaaactttgaacagcctcacagagcagaagactctgtgcaccgagttgaccgtaac
agacatctttgctgcctccaagaacacaactgagaaggaaaccttctgcagggctgcgactgtgctccggcagttctacag
ccaccatgagaaggacactcgctgcctgggtgcgactgcacagcagttccacaggcacaagcagctgatccgattcctga
aacggctcgacaggaacctctggggcctggcgggcttgaattcctgtcctgtgaaggaagccaaccagagtacgttggaa
aacttcttggaaaggctaaagacgatcatgagagagaaatattcaaagtgttcgagctga
SEQ ID NO: 11 shows the nucleotide sequence of human IL-7. 
Atgttccatgtttcttttaggtatatctttggacttcctcccctgatccttgttctgttgccagtagcatcatctgattgtga
tattgaaggtaaagatggcaaacaatatgagagtgttctaatggtcagcatcgatcaattattggacagcatgaaagaaattg
gtagcaattgcctgaataatgaatttaacttttttaaaagacatatctgtgatgctaataaggaaggtatgtttttattccgtgctgc
tcgcaagttgaggcaatttcttaaaatgaatagcactggtgattttgatctccacttattaaaagtttcagaaggcacaacaata
ctgttgaactgcactggccaggttaaaggaagaaaaccagctgccctgggtgaagcccaaccaacaaagagtttggaaga
aaataaatctttaaaggaacagaaaaaactgaatgacttgtgtttcctaaagagactattacaagagataaaaacttgttggaa
taaaattttgatgggcactaaagaacactga
SEQ ID NO: 12 shows the nucleotide sequence of human IL-9. 
**atgcttctggccatggtccttacctctgccctgctcctgtgctccgtggcaggc**caggggtgtccaaccttggcggggat
cctggacatcaacttcctcatcaacaagatgcaggaagatccagcttccaagtgccactgcagtgctaatgtgaccagttgt
ctctgtttgggcattccctctgacaactgcaccagaccatgcttcagtgagagactgtctcagatgaccaataccaccatgca
aacaagatacccactgattttcagtcgggtgaaaaaatcagttgaagtactaaagaacaacaagtgtccatatttttcctgtga
acagccatgcaaccaaaccacggcaggcaacgcgctgacatttctgaagagtcttctggaaattttccagaaagaaaagat
gagagggatgagaggcaagatatga
SEQ ID NO: 13 shows the nucleotide sequence of human IL-15. 
atgagaatttcgaaaccacatttgagaagtatttccatccagtgctacttgtgtttacttctaaacagtcattttctaact
gaagctggcattcatgtcttcattttgggctgtttcagtgcagggcttcctaaaacagaagccaactgggtgaatgtaataagt
gatttgaaaaaaattgaagatcttattcaatctatgcatattgatgctactttatatacggaaagtgatgttcaccccagttgcaaa
gtaacagcaatgaagtgctttctcttggagttacaagttatttcacttgagtccggagatgcaagtattcatgatacagtagaaa
atctgatcatcctagcaaacaacagtttgtcttctaatgggaatgtaacagaatctggatgcaaagaatgtgaggaactggag
gaaaaaaatattaaagaatttttgcagagttttgtacatattgtccaaatgttcatcaacacttcttga
SEQ ID NO: 14 shows the nucleotide sequence of human IL-21. 
atgagatccagtcctggcaacatggagaggattgtcatctgtctgatggtcatcttcttggggacactggtccacaaa
tcaagctcccaaggtcaagatcgccacatgattagaatgcgtcaacttatagatattgttgatcagctgaaaaattatgtgaat
gacttggtccctgaatttctgccagctccagaagatgtagagacaaactgtgagtggtcagctttttcctgctttcagaaggcc
caactaaagtcagcaaatacaggaaacaatgaaaggataatcaatgtatcaattaaaaagctgaagaggaaaccaccttcc
acaaatgcagggagaagacagaaacacagactaacatgcccttcatgtgattcttatgagaaaaaaccacccaaagaattc
ctagaaagattcaaatcacttctccaaaagatgattcatcagcatctgtcctctagaacacacggaagtgaagattcctga
SEQ ID NO: 15 shows the nucleotide sequence of human IL-25. 
Atgagggagcgacccagattaggtgaggacagttctctcattagccttttcctacaggtggttgcattcttggcaatg
gtcatggaacccacacctacagccactggcccagctgctgccccagcaaagggcaggacacctctgaggagctgctg
 
aggtggagcactgtgcctgtgcctcccctagagcctgctaggcccaaccgccacccagagtcctgtagggccagtgaag
atggacccctcaacagcagggccatctccccctggagatatgagttggacagagacttgaaccggctcccccaggacctg
taccacgcccgttgcctgtgcccgcactgcgtcagcctacagacaggctcccacatggacccccggggcaactcggagc
tgctctaccacaaccagactgtcttctaccggcggccatgccatggcgagaagggcacccacaagggctactgcctggag
cgcaggctgtaccgtgtttccttagcttgtgtgtgtgtgcggccccgtgtgatgggctag
SEQ ID NO: 16 shows the nucleotide sequence of human IL-33, in which shown in bold is the nucleotide sequence comprised in the propeptide. 
atgaagcctaaaatgaagtattcaaccaacaaaatttccacagcaaagtggaagaacacagcaagcaaagccttg
tgtttcaagctgggaaaatcccaacagaaggccaaagaagtttgccccatgtactttatgaagctccgctctggcctt
atgataaaaaggaggcctgttactttaggagagaaaccaccaaaggccttcactgaaaacaggtagaaagca
caaaagacatctggtactcgctgcctgtcaacagcagtctactgtggagtgctttgcctttggtatatcaggggtccaga
aatatactagagcacttcatgattcaagtatcacaggaatttcacctattacagagtatcttgcttctctaagcacatacaatgat
caatccattacttttgctttggaggatgaaagttatgagatatatgttgaagacttgaaaaaagatgaaaagaaagataaggtg
ttactgagttactatgagtctcaacacccctcaaatgaatcaggtgacggtgttgatggtaagatgttaatggtaaccctgagt
cctacaaaagacttctggttgcatgccaacaacaaggaacactctgtggagctccataagtgtgaaaaaccactgccagac
caggccttctttgtccttcataatatgcactccaactgtgtttcatttgaatgcaagactgatcctggagtgtttataggtgtaaag
gataatcatcttgctctgattaaagtagactcttctgagaatttgtgtactgaaaatatcttgtttaagctctctgaaacttag
SEQ ID NO:17 shows the amino acid sequence of the exemplary HSA-IL-7 fusion protein, in which the linker region GGGGS is shown in underlined and human IL-7 is fused to the C-terminal of human serum albumin. 
DAHKSEVAHRFKDLGEENFKALVLIAFAQYLQQCPFEDHVKLVNEVTEFAKT
CVADESAENCDKSLHTLFGDKLCTVATLRETYGEMADCCAKQEPERNECFL
QHKDDNPNLPRLVRPEVDVMCTAFHDNEETFLKKYLYEIARRHPYFYAPELL
FFAKRYKAAFTECCQAADKAACLLPKLDELRDEGKASSAKQRLKCASLQKF
GERAFKAWAVARLSQRFPKAEFAEVSKLVTDLTKVHTECCHGDLLECADDR
ADLAKYICENQDSISSKLKECCEKPLLEKSHCIAEVENDEMPADLPSLAADFV
ESKDVCKNYAEAKDVFLGMFLYEYARRHPDYSVVLLLRLAKTYETTLEKCC
AAADPHECYAKVFDEFKPLVEEPQNLIKQNCELFEQLGEYKFQNALLVRYTK
KVPQVSTPTLVEVSRNLGKVGSKCCKHPEAKRMPCAEDYLSVVLNQLCVLH
EKTPVSDRVTKCCTESLVNRRPCFSALEVDETYVPKEFNAETFTFHADICTLSE
KERQIKKQTALVELVKHKPKATKEQLKAVMDDFAAFVEKCCKADDKETCFA
EEGKKLVAASQAALGLGGGGSGGGGSGGGGSDCDIEGKDGKQYESVLMVSI
DQLLDSMKEIGSNCLNNEFNFFKRHICDANKEGMFLFRAARKLRQFLKMNST
GDFDLHLLKVSEGTTILLNCTGQVKGRKPAALGEAQPTKSLEENKSLKEQKK
LNDLCFLKRLLQEIKTCWNKILMGTKEH
SEQ ID NO:18 shows the amino acid sequence of the exemplary IL-25-HSA fusion protein, in which the linker region GGGGS is shown in underlined and human IL-25 is fused to the N-terminal of human serum albumin. 
YSHWPSCCPSKGQDTSEELLRWSTVPVPPLEPARPNRHPESCRASEDGPLNSR
AISPWRYELDRDLNRLPQDLYHARCLCPHCVSLQTGSHMDPRGNSELLYHNQ
TVFYRRPCHGEKGTHKGYCLERRLYRVSLACVCVRPRVMGGGGGSGGGGSG
GGGSDAHKSEVAHRFKDLGEENFKALVLIAFAQYLQQCPFEDHVKLVNEVTE
FAKTCVADESAENCDKSLHTLFGDKLCTVATLRETYGEMADCCAKQEPERN
 
ECFLQHKDDNPNLPRLVRPEVDVMCTAFHDNEETFLKKYLYEIARRHPYFYA
PELLFFAKRYKAAFTECCQAADKAACLLPKLDELRDEGKASSAKQRLKCASL
QKFGERAFKAWAVARLSQRFPKAEFAEVSKLVTDLTKVHTECCHGDLLECA
DDRADLAKYICENQDSISSKLKECCEKPLLEKSHCIAEVENDEMPADLPSLAA
DFVESKDVCKNYAEAKDVFLGMFLYEYARRHPDYSVVLLLRLAKTYETTLE
KCCAAADPHECYAKVFDEFKPLVEEPQNLIKQNCELFEQLGEYKFQNALLVR
YTKKVPQVSTPTLVEVSRNLGKVGSKCCKHPEAKRMPCAEDYLSVVLNQLC
VLHEKTPVSDRVTKCCTESLVNRRPCFSALEVDETYVPKEFNAETFTFHADIC
TLSEKERQIKKQTALVELVKHKPKATKEQLKAVMDDFAAFVEKCCKADDKE
TCFAEEGKKLVAASQAALGL
SEQ ID NO:19 shows the amino acid sequence of the exemplary IgG4mFc-IL-7 fusion protein, in which the linker region GGGGS is shown in underlined and human IL-7 is fused to the C-terminal of human IgG4m Fc. 
ESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVM
HEALHNHYTQKSLSLSLGKGGGGSGGGGSGGGGSDCDIEGKDGKQYESVLM
VSIDQLLDSMKEIGSNCLNNEFNFFKRHICDANKEGMFLFRAARKLRQFLKM
NSTGDFDLHLLKVSEGTTILLNCTGQVKGRKPAALGEAQPTKSLEENKSLKEQ
KKLNDLCFLKRLLQEIKTCWNKILMGTKEH
SEQ ID NO:20 shows the amino acid sequence of the exemplary IgG4mFc-IL-25 fusion protein, in which the linker region GGGGS is shown in underlined and human IL-25 is fused to the C-terminal of human IgG4m Fc. 
ESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVM
HEALHNHYTQKSLSLSLGKGGGGSGGGGSGGGGSYSHWPSCCPSKGQDTSE
ELLRWSTVPVPPLEPARPNRHPESCRASEDGPLNSRAISPWRYELDRDLNRLP
QDLYHARCLCPHCVSLQTGSHMDPRGNSELLYHNQTVFYRRPCHGEKGTHK
GYCLERRLYRVSLACVCVRPRVMG
SEQ ID NO: 21 is uPA (the substrate sequence of urokinase-type plasminogen activator) sensitive cleavage sequence, and ↓ shows the cleavage site.
   LSGR↓SDNH
SEQ ID NO: 22 is legumain (cysteine protease) sensitive cleavage sequence, and ↓ shows the cleavage site.
   AAN↓L
SEQ ID NO: 23 is legumain (cysteine protease) sensitive cleavage sequence, and ↓ shows the cleavage site.
   AAN↓V
SEQ ID NO: 24 is MMP-1 sensitive cleavage sequence, and ↓ shows the cleavage site.
   PLG↓LWA
SEQ ID NO: 25 is MMP-2/9 sensitive cleavage sequence, and ↓ shows the cleavage site.
   PAA↓LVGA
SEQ ID NO: 26 is MMP-14 sensitive cleavage sequence, and ↓ shows the cleavage site.
   SGRIGF↓LRTA
SEQ ID NO: 32 is MMP-14 sensitive cleavage sequence, and ↓ shows the cleavage site.
   PAG↓LVG
SEQ ID NO: 27 is Cathepsin B sensitive cleavage sequence, and ↓ shows the cleavage site.
   GFLG↓
SEQ ID NO: 28 is glycin-serine linker.
   (GGGGS)ₙ, n=1-3
SEQ ID NO: 29 is α-helix forming peptide linker.
   (EAAAK)ₙ, n=2-5
SEQ ID NO: 30 shows the amino acid sequence of the exemplary IL-25+IL7 recombinant protein, in which the sequence of the uPA cleavable peptide linker between two active components is shown in bold, uPA recognition substrate sequence is shown in single-underlined, and tumor penetrating peptide iRGD is shown in double-underlined. 
YSHWPSCCPSKGQDTSEELLRWSTVPVPPLEPARPNRHPESCRASEDGPLNSR
AISPWRYELDRDLNRLPQDLYHARCLCPHCVSLQTGSHMDPRGNSELLYHNQ
TVFYRRPCHGEKGTHKGYCLERRLYRVSLACVCVRPRVMGGGSGLSGRSDN
HGGGGSDCDIEGKDGKQYESVLMVSIDQLLDSMKEIGSNCLNNEFNFFKRHI
CDANKEGMFLFRAARKLRQFLKMNSTGDFDLHLLKVSEGTTILLNCTGQVK
GRKPAALGEAQPTKSLEENKSLKEQKKLNDLCFLKRLLQEIKTCWNKILMGT
KEHGGGGSGGGGSGGGGSCRGDKGPDC
SEQ ID NO: 31 shows the amino acid sequence of the exemplary IL-25+IL-7+IL-2 recombinant protein, in which the sequence of the uPA cleavable peptide linker between active components is shown in bold, uPA substrate sequence is shown in single-underlined, and tumor penetrating peptide iRGD is shown in double-underlined.

YSHWPSCCPSKGQDTSEELLRWSTVPVPPLEPARPNRHPESCRASEDGPLNSR

AISPWRYELDRDLNRLPQDLYHARCLCPHCVSLQTGSHMDPRGNSELLYHNQ
TVFYRRPCHGEKGTHKGYCLERRLYRVSLACVCVRPRVMGGGSGLSGRSDN
HGGGGSDCDIEGKDGKQYESVLMVSIDQLLDSMKEIGSNCLNNEFNFFKRHI
CDANKEGMFLFRAARKLRQFLKMNSTGDFDLHLLKVSEGTTILLNCTGQVK
GRKPAALGEAQPTKSLEENKSLKEQKKLNDLCFLKRLLQEIKTCWNKILMGT
KEHGGGSGLSGRSDNHGGGGSAPTSSSTKKTQLQLEHLLLDLQMILNGINN
YKNPKLTAMLTKKFYMPKKATELKHLQCLEEELKPLEEVLNLAQSKNFHLRP
RDLISNINVIVLELKGSETTFMCEYADETATIVEFLNRWITFCQSIISTLTGGGGS
GGGGSGGGGSCRGDKGPDC

### DETAILED DESCRIPTION OF EMBODIMENTS

Unless otherwise stated, each term in the present disclosure has the same meaning as commonly understood by one of ordinary skill in the art.

As used herein, the terms "γc-cytokine" or "γc-cytokine family" and "common cytokine receptor gamma-chain family" are used interchangeably and refer to the family of cytokines composed of six members (i.e., IL-2, IL-4, IL-7, IL-9, IL-15 and IL-21), all members of which signal through a receptor complex containing a common cytokine receptor gamma chain subunit.

In one embodiment, typical suitable γc-cytokines include the mature portions in which the respective signal peptide sequences are removed or the active portions in which internal partial amino acids are deleted. For example, IL-7 includes but is not limited to the mature IL-7 polypeptide portion (the signal peptide sequence in the sequence as set forth in SEQ ID NO: 3 is removed), or includes sequences in which some of the amino acids at positions 96 to 114 of SEQ ID NO: 3 are deleted.

As used herein, "IL-25", also known as IL-17E, is a member of the structurally related IL-17 family but is functionally distinct from other IL-17 family members. A typical suitable IL-25 includes, but is not limited to, the mature IL-25 polypeptide portion (the signal peptide sequence in the sequence as set forth in SEQ ID NO: 7 is removed).

As used herein, "IL-33" is a new member of the IL-1 family. A typical suitable IL-33 includes, but is not limited to, the mature portion of the IL-33 polypeptide (the bold sequence in the sequence as set forth in SEQ ID NO: 16 is removed).

In the present disclosure, reference to the amino acid sequences of various cytokines and the nucleotide sequences encoding the same encompasses their corresponding variants or mutants. Various variants or mutants of cytokines known in the art can be used herein, and can be found at http://www.ncbi.nlm.nih.gov/. Generally, a variant of a particular nucleotide sequence will have at least about 70%, 75%, 80%, 85%, 86%, 87%, 88%, 89%, 90%, 91%, 92%, 93%, 94%, 95%, 96%, 97%, 98%, 99%, 99.5% or 99.9% or higher sequence identity with the particular nucleotide sequence, or be complementary sequences of the above. Such variant sequences include additions, deletions or substitutions of one or more nucleic acid residues, which may result in the addition, removal or substitution of corresponding amino acid residues. Sequence identity is determined by sequence alignment programs known in the art, including hybridization techniques.

Thus, nucleotide sequence variants can differ from the indicated sequences by as few as 1-15 nucleotides, as few as 1-10 (e.g., 6-10), as few as 5, as few as 4, 3, 2 or even 1 nucleotide. Accordingly, amino acid sequence variants can differ from the indicated sequences by as few as 1-15 amino acids, as few as 1-10 (eg, 6-10), as few as 5, as few as 4, 3, 2 or even 1 amino acid.

In one embodiment, taking IL-7 as an example, its variants may be active variants having at least 70%, 75%, 80%, 85%, 86%, 87%, 88%, 89%, 90%, 91%, 92%, 93%, 94%, 95%, 96%, 97%, 98%, 99%, 99.5% or 99.9% or higher identity with the amino acid sequence as set forth in SEQ ID NO:3. Correspondingly, the variants of the nucleic acid encoding IL-7 may be those having at least 70%, 75%, 80%, 85%, 86%, 87%, 88%, 89%, 90%, 91%, 92%, 93%, 94%, 95%, 96%, 97%, 98%, 99%, 99.5% or 99.9% or higher identity with the nucleotide sequence as set forth in SEQ ID NO: 11.

In certain embodiments, to extend the *in vivo* half-life of a peptide or proteinaceous drug, and to improve its pharmacokinetic and pharmacodynamic profile, the various cytokines provided herein may also have any suitable chemical modifications known in the art, e.g., PEGylation, Xtenylation, PASylation, Hesylation or fusion with other proteins. The various cytokines provided herein may also have or have no a tag, including but not limited to any subtypes or variants of human antibody Fc, human serum albumin (HSA) or human transferrin, and these tags are fused to the N-terminal or C- terminal of cytokines.

Specifically, Xtenylation involves an XTEN technology recently developed by Amunix, Inc., which relies on the peptide chains of different lengths without specific conformation consisting of six amino acids, Ala, Glu, Gly, Pro, Ser, and Thr, termed as XTEN sequences. When linked to the peptide or proteinaceous drug via a gene fusion method, it increases the solubility and stability of the drug, and is characterized by low immunogenicity, high product purity, and complete biodegradation. In addition, XL-protein Inc. uses a random coiled protein structure constructed by repeated Pro, Ala, and Ser. This technique, called PASylation, uses these three amino acids to form a PAS sequence of 200-600 amino acid residues. Linking the PAS sequence to proteinaceous or peptide drugs via a gene fusion method, increases the volume, thereby preventing rapid filtration of the kidney and prolonging the half-life.

In one embodiment, with the IL-7 and IL-25 as an example, the amino acid sequences of their human serum albumin (HSA) fusion proteins are shown in SEQ ID NOs: 17 and 18, respectively, and the amino acid sequences of the IgG4mFc fusion proteins are shown in SEQ ID NOs: 19 and 20, respectively.

In certain embodiments, in order to enhance the enrichment of a protein of interest in a tumor, a tumor-penetrating peptide can be fused to the N-terminal or C-terminal of the cytokines, including but not limited to fusion of cRGD peptide or cNGR at its N-terminal, fusion of iRGD peptide or iNGR peptide or pHLIPs peptide at its C-terminal; a tumor-targeted single-chain or single-domain antibody can be fused to the N-terminal or C-terminal of cytokines, including but not limited to fusion with single-chain or single-domain antibody targeting Tn-Muc1, EDB-Fibronectin, mesothelin, FAP, CEA, HER2, GD2, PD-L1 or EGFR.

In certain embodiments, in order to simplify the dosage form, process, and the convenience of application of a combination drug, the various combinations of cytokines provided herein can be achieved by fusion proteins, and the peptide linker which are cleavable *in vivo* or intratumorally can be comprised between the active components of the recombinant protein.

In one embodiment, with the IL-7 and IL-25 as an example, the amino acid sequence of the recombinant protein of IL-25 and IL-7 comprising the uPA cleavable linker and the tumor penetrating peptide iRGD is set forth in SEQ ID NO: 30.

In one embodiment, with the IL-7, IL-25 and IL-2v variants (R38A/F42K) as an example, the amino acid sequence of the recombinant protein of IL-25, IL-7 and IL-2v comprising the uPA cleavable linker and the tumor penetrating peptide iRGD is set forth in SEQ ID NO:31.

As used herein, the term "recombinant protein" refers to a specific recombinant protein molecule which is expressed by the use of genetic recombination techniques known in the art to obtain a recombinant vector linked to a gene fragment that can be translated into a protein of interest, which is then transformed into a host cell which expresses the protein of interest.

In one embodiment, the recombinant protein provided herein includes, but is not limited to, one or more selected from the group consisting of yc-cytokine family members, such as IL-2, IL-4, IL-7, IL-9, IL-15 or IL-21, and IL-25 or IL-33.

In another embodiment, the referred nucleic acid coding sequences of these cytokines herein may be present in separate vectors; or the nucleic acid coding sequences of these cytokines are present in the same vector in combinations of any two or three.

Further, the recombinant protein provided herein can be expressed by different cells or populations of cells, or by the same cell or population of cells.

Alternatively, the recombinant nucleic acid molecule or vector provided herein can be included in different cells or population of cells, or can be included in the same cell or population of cells.

As used herein, the term "vector" refers to any recombinant polynucleotide that can be used to introduce heterologous DNA into a host cell to result in phenotype transformation. One type of vector is a plasmid that involves a circular double-stranded nucleic acid into which other deoxyribonucleic acid fragments can be inserted. Another type of vector is a viral vector in which an exogenous DNA fragment can be inserted into a viral genome, and transcripts can be packaged into infectious viral particles and transduced into target cells. Upon transduction, some viral vectors, such as retroviral or lentiviral vectors, can be integrated into the host cell genome and replicate together with the host genome.

Furthermore, vectors capable of directing gene expression are referred to as "expression vectors". In particular, an expression vector is capable of replicating and expressing a gene of interest in a transformed or transfected host cell. The expression vector can include one or more phenotypic selection markers and a replication origin sequence to ensure amplification of the maintained vector within organism's cells. Expression vectors also include the expression of a promoter-driven polypeptide in a cell. A suitable expression vector can be a plasmid or a viral vector.

As used herein, the term "prevention and/or treatment" includes preventing, inhibiting, curing, alleviating or ameliorating a malignant tumor, as well as preventing or delaying the metastasis of primary cancer.

In certain embodiments, the malignant tumors that are prevented and/or treated include malignant tumors at various stages, such as sarcomas and carcinomas, including but not limited to the following types: fibrosarcoma, mucinous sarcoma, liposarcoma, chondrosarcoma, osteosarcoma , chordoma, angiosarcoma, endothelial sarcoma, lymphangiosarcoma, lymphangial endothelial sarcoma, synovial sarcoma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous gland cancer, papillary carcinoma, papillary adenocarcinoma, bronchial carcinoma, medullary carcinoma, renal cell carcinoma, liver cancer, cholangiocarcinoma, choriocarcinoma, seminoma, embryonic carcinoma, nephroblastoma, cervical cancer, testicular tumor, lung cancer, small cell lung cancer, bladder cancer, epithelial cancer, glioma, astrocytoma, medulloblastoma, craniopharyngioma, ependymoma, pineal tumors, hemangioblastoma, acoustic neuroma, meningioma, oligodendroglioma, melanoma, neuroblastoma, retinoblastoma.

In one embodiment, the sarcoma that is prevented and/or treated is such as fibrosarcoma, mucinous sarcoma, liposarcoma, chondrosarcoma, osteosarcoma, chordoma, angiosarcoma, endothelial sarcoma, lymphangiosarcoma, lymphangial endothelial sarcoma, synovial sarcoma, mesothelioma, Ewing's tumor, leiomyosarcoma, rhabdomyosarcoma, etc.

In another embodiment, the cancer that is prevented and/or treated is such as colon cancer, pancreatic cancer, breast cancer, ovarian cancer, prostate cancer, squamous cell carcinoma, basal cell carcinoma, adenocarcinoma, sweat gland cancer, sebaceous gland cancer, papillary carcinoma, papillary adenocarcinoma, bronchial carcinoma, medullary carcinoma, renal cell carcinoma, liver cancer, cholangiocarcinoma, choriocarcinoma, seminoma, embryonal carcinoma, nephroblastoma, cervical cancer, testicular tumor, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, meningioma, oligodendroglioma, melanoma, neuroblastoma, retinoblastoma, etc.

In yet another embodiment, the advanced cancer that is prevented and/or treated includes malignant melanoma, lung cancer, colon cancer, colon cancer, liver cancer, head and neck cancer, sarcoma, prostate cancer, and advanced cancer which has developed to a certain stage and is unable to be cured by conventional therapy.

As used herein, the term "combination therapy" refers to the administration of the combination of at least one of γc-cytokine or an active part or variant thereof and at least one of IL-25 or IL-33 or an active part or variant thereof to a subject in need thereof. Combination therapy can provide "synergistic" and "synergistic effects", i.e., the effect achieved when the active ingredients are used together is higher than the sum of the effects achieved when the compounds are used separately. When the active ingredients are: (1) co-prepared and administered, or simultaneously delivered in the form of a combined preparation; (2) as separate preparations, delivered alternately or in parallel; or (3) by some other means, synergistic effects can be achieved.

For example, in one embodiment, the combinations provided herein comprise or consist of: i) any one, two, three, four, five or six of interleukins selected from the group consisting of IL-2, IL-4, IL-7, IL-9, IL-15 and IL-21; and ii) any one or two of interleukins selected from the group consisting of IL-25 and IL-33.

In a specific embodiment, the combinations provided herein comprise or consist of: i) any one or two of interleukins selected from the group consisting of IL-2, IL-4, IL-7, IL-9, IL-15 and IL-21; and ii) any one of interleukins selected from the group consisting of IL-25 and IL-33.

In another embodiment, the combinations provided herein comprise or consist of: i) any one or two interleukins selected from the group consisting of IL-7, IL-9 and IL-15; and ii) any one of interleukins selected from the group consisting of IL-25 and IL-33.

In still another embodiment, the combinations provided herein comprise or consist of: i) any one or two of interleukins selected from the group consisting of IL-7, IL-9 and IL-15; and ii) IL -25.

Further, in one embodiment, the combinations provided herein comprise or consist of: i) IL-7; and ii) any one of interleukins selected from the group consisting of IL-25 and IL-33.

Still further, in another embodiment, the combinations provided herein comprise or consist of: i) IL-7; ii) IL-25; and iii) any one of interleukins selected from the group consisting of IL-2, IL-9 and IL-15.

In certain embodiments, a combination comprising three interleukins exhibits more potent inhibitory and less time-consuming effect on tumors as compared to a combination comprising two interleukins.

In particular embodiments, the interleukin combinations provided herein can be administered to an individual simultaneously or sequentially.

In a specific embodiment, the interleukin combination provided herein can be administered intravenously or subcutaneously or intraperitoneally or intramuscularly to an individual.

As used herein, the term "therapeutically effective amount" can be determined on a case-by-case basis, and can be readily grasped by one of ordinary skill in the art based on the actual amount of drug required, such as being determined based on the patient's weight, age, and condition. Since extracts are all non-toxic ingredients, they can be administered directly as desired, in which the composition does not contain a pharmaceutically acceptable carrier. When a composition contains a pharmaceutically acceptable carrier, they can be mixed in a conventional manner in the pharmaceutical field to prepare a desired drug.

Hereinafter, specific embodiments of the combination therapy herein will be described by taking a combination of IL-7 and IL-25 as an example.

In one embodiment, a combination of IL-7 and IL-25 or a combination of active mutants thereof is administered to a mammalian host. In another embodiment, in addition to the purified recombinant proteins, IL-7 and IL-25 may also be derived from genetically engineered cells, including but not limited to any transformed cells, autologous cells such as dendritic cells or T cells, which can be transformed by viral vectors to express active IL-7 or IL-25 or both, and can be applied to an organism. In yet another embodiment, the transformed cells or cell lines can be encapsulated in a hydrogel such as a sodium alginate gel, thereby being applied to an organism.

In one embodiment, the preferred dose of the IL-7 and IL-25 recombinant protein for individual treatment is 0.0001-10 mg/kg body weight/day. In another embodiment, upon microencapsulation of an engineered cell line expressing IL-7 or IL-25, the preferred dose for an organism is between 1 × 10⁶ and 1 × 10¹².

In certain embodiments, the combination of the cytokines IL-7 and IL-25 as described herein can be obtained by mixing IL-7- and IL-25-producing cells. In a specific embodiment, microencapsulated engineered cells that simultaneously produce both cytokines of IL-7 and IL-25 can be injected subcutaneously or intraperitoneally into a host.

In certain embodiments, the combination of the cytokines IL-7 and IL-25 as described herein can be delivered by injecting a recombinant fusion protein comprising an intratumorally cleavable peptide between the IL-7 and IL-25 molecules. In a specific embodiment, the recombinant protein can be injected intravenously or intraperitoneally or intratumorally into a host.

When engineered autologous dendritic cells capable of simultaneously secreting both IL-7 and IL-25 are used as a combination therapy, the preferred dose is between 1 ×10⁶ and 1×10¹² cells, and these cells can be injected subcutaneously or intramuscularly into a host. Similarly, if the cells used are T cells, the same dose can be used, but T cells are injected intravenously into a host.

The following examples are only for illustrative purposes and not intended to limit the scope of the application. Unless otherwise indicated, the examples are performed according to routine experimental conditions, such as the Sambrook J & Russell DW, Molecular cloning: a laboratory manual, 2001, or those as suggested by the manufacturer's instructions.

### Examples

### Example 1

In this example, IL-7 is taken as an example to provide a method for preparing a tumor cell pool for producing cytokines, which specifically comprises the following steps:
In the first step, the cDNA of the mouse cytokine IL-7 containing the coding sequence and the signal peptide was synthesized from a self-synthesized mouse spleen and lung reverse transcription reaction products (manufactured by the reverse transcription reaction kit of Thermo Scientific Inc.) by PCR.

In the second step, these genes were separately cloned into the retroviral vector pBabe-SV40-puro (purchased from http://www.addgene.org/) and the resultant sequences were confirmed.

In the third step, human embryonic kidney HEK293T cells (purchased from http://www.atcc.org/) were co-transfected with pCL-Ampho helper plasmid (purchased from NOVUS Biologicals Inc.) and pBabe-derived plasmid expressing cytokines to produce a retrovirus.

Briefly, the medium of HEK293T cells plated in p100 cell culture dishes on the previous day was replaced with pre-warmed pen/strp-free DMEM-10 medium (DMEM containing 10% fetal calf serum and glutamine) 10 min prior to transient transfection. 5 µg of pCL-Ampho helper plasmid and 5 µg of pBabe-derived plasmid were mixed in an Eppendof tube, and then added 1 ml of Opti-MEM medium and 30 µl of PEI transfection reagent. After incubation for 10 minutes at room temperature, the DNA pellet-containing mixture was added to the culture dish of the above HEK-293T cells. After 6 hours, Pen/strp was added overnight, and then replaced with new DMEM-10 medium. After 24 hours, the supernatant containing the retrovirus was collected and sterilized through a 45 µM filter.

In the fourth step, mouse melanoma B16F10 cells (purchased from http://www.atcc.org/) were transduced with retrovirus, and a tumor cell pool producing cytokine IL-7 was obtained after enrichment.

Briefly, B16F10 cells cultured in p100 cell culture dishes were transduced overnight with the resultant retrovirus plus polybrene (final concentration of 8 µg/mL), and then the culture medium was changed to DMEM10 medium and puromycin (final concentration of 3 µg / mL) was added to enrich cells transduced by viruses. The cells were expanded and frozen in a cryopreservation solution for long-term storage.

Similarly, tumor cell pools comprising other cytokines, such as IL-1b, IL-2, IL-4, IL-5, IL-8, IL-9, IL-13, IL-15, IL-17A, IL-18, IL-21, IL-25, IL-28, IL-36, can be produced. Furthermore, the above two or more cell pools can be pooled to produce a tumor cell pool that co-expresses two or more cytokines.

### Example 2

Upon the preparation of a tumor cell pool for producing cytokines, in this example, combination of cytokines which synergistically inhibits tumor growth is further screened by *in vivo* experiments.

Experimental animals: Female C57BL/6 mice, approximately 2-3 months old, were shaved on both sides to facilitate injection to tumor cells and measurement of tumor size.

Cell preparation: B16F10 primary cells that did not grow to complete confluence were trypsinized, and the cell concentration was determined by a hemocytometer.

*In vivo* experiments: 5×10⁵ B16F10 primary cells suspended in RPMI-1640 medium were injected intradermally (i.d.) into the left of female C57BL/6 mice as response tumors. Similarly, a tumor cell pool expressing specific cytokines or combinations thereof (each 1 x 10⁶ cells) were injected intradermally into the right as cytokine tumors. Mice were sacrificed on day 16 and tumor size was measured.

"cytokine tumor" used herein is a drug-sustaining generator that will continuously release cytokines to activate the body's immune system. The activated immune system will kill the cytokine tumor itself and response tumors, while the size of "effect tumors" reflects the actual therapeutic effect of the combination drug. The drug combination for the inhibition of unilateral tumors (mostly for cytokine tumors themselves) indicates that the combination requires a relatively high concentration of cytokines or has the effect of stimulating immune cells resident in local tissues to inhibit tumors, or the combination has strong chemotaxis-promoting effect and can be used as a control. A combination exhibiting significant inhibitory effect on both sides of tumors is considered to be a therapeutically valuable combination.

The experimental flow diagram of the above-described preparation of a cytokine-producing tumor cell pool and verification of tumor growth inhibition is shown in Fig. 1.

Fig. 2 shows exemplary results demonstrating tumor growth inhibition using different cytokine-producing tumor cell pools or combinations thereof.

### Example 3

This example utilizes a pool of tumor cells producing the cytokines IL-7 and IL-25 to treat tumors, as follows.

Female C57BL/6 mice of 2-3 month old were divided into the following 4 groups with 10 per group:
Control group, 5×10⁵ B16F10 primary cells were injected intradermally on both sides;
IL-7 treatment group, the IL-7-producing cell pool (1 million) was injected into the right side of the mouse, and the B16F10 primary cell was injected into the left side (5×10⁵ as an response tumor);
IL-25 treatment group, the IL-25-producing cell pool was intradermally injected into the right side, and the B16F10 primary cells were intradermally injected into the left side;
IL-7 + IL-25 treatment group, 1 million of each IL-7- and IL-25-producing cell pool were intradermally injected into the right side, and the B16F10 primary cells were intradermally injected into the left side.

After 4 days, the tumor area on both sides was measured daily using a vernier caliper.

The experimental results are shown in Fig. 3.

In addition, the cell pool co-expressing hu-IL-7 and hu-IL-25 obtained the same result.

### Example 4

This example provides expression and purification of the recombinant hu-IL-7 and hu-IL-25 proteins, and the therapeutic effects thereof on tumors.

### 1. Expression and purification of recombinant hu-IL-7 or hu-IL-25 protein

The 6×His N-terminally labeled human IL-7 or IL-25 cDNA non-signal peptide region coding sequence was synthesized by PCR and cloned into the pET15 plasmid vector (purchased from Novagen Inc.).

After sequence verification, the vector was transformed into *E. coli* BL21 (DE3) (purchased from NEB Inc.), and the transformed single colonies were inoculated into 10 ml of LB medium (containing 100 µg/ml ampicillin) and cultured overnight on a shaker at 250 rpm/min at 37°C. 5 ml of the initial culture was transferred to 500 ml of ampicillin-containing LB medium, and cultured on a shaker at 25°C until the OD600 reached about 0.4. IPTG was added to 200 µM to induce culture overnight.

The obtained culture was centrifuged and resuspended in suspension buffer (containing 20 mM HEPES pH 7.5, sodium chloride 50 mM, 0.5 mM EDTA, a protease inhibitor). The cells were disrupted by ultrasonic wave, and the supernatant was removed after high speed centrifugation. The precipitate containing the inclusion body was washed once with a suspension buffer, and then dissolved in a denaturing buffer (50 mM Tris-HCl pH 7.5, 500 mM NaCl, 6 M guanidine hydrochloride). Upon centrifugation at high speed, the supernatant contained denatured inclusion body proteins.

The resulted supernatant was passed through a 1.5 ml Ni-NTA agarose affinity chromatography column to further purify the denatured proteins. The column was washed with 20 ml of denaturing buffer, and then washed with 10 ml of denaturing buffer containing 10 mM imidazole. The recombinant protein was eluted with 10 ml of denaturing buffer containing 0.1 M imidazole.

The protein renaturation procedure was subsequently performed. The eluted mixed proteins were dialyzed with dialysis buffer A (50 mM Tris-HCl pH8.0, sodium chloride 50 mM, 1 mM EDTA, 10 mM DTT, 6 M guanidine hydrochloride) for 24 hours at 4°C. They were then dialyzed overnight with dialysis buffer A without DTT. The proteins were adjusted to 0.2 mg/ml of concentration, and then dialyzed with renaturation buffer (50 mM Tris-HCl pH 8.0, 100 mM arginine, 5 mM reduced glutathione, 0.5 mM oxidized glutathione, 50 mM NaCl) for 3 days at 4 °C with dialysate changing daily. The proteins were then dialyzed overnight with glutathione-free renaturation buffer, and finally dialyzed with 1x PBS for one day. The renatured protein solution was centrifuged to remove residual aggregates. Residual trace amount of LPS was removed through a 1 ml polymyxin B agarose column. The proteins were concentrated by ultrafiltration. The purified protein was subpackaged and stored.

The protein concentration was determined by the Bradford method. 10 µg of the partially purified protein was subjected to 4-20% SDS PAG gradient gel electrophoresis, and stained with Coomassie brilliant blue. The results are shown in Fig. 4A and Fig. 4B.

### 2. Treatment of tumors with the combination of purified IL-7 and IL-25 proteins

Tumors were treated with the combination of purified IL-7 and IL-25 proteins. If the hosts had used antibiotics, the administration of all antibiotics should be terminated at least one of week prior to treatment and during treatment, as antibiotic administration will severely affect the efficacy of combination therapy or make treatment completely fail. C57BL/6 mice were divided into 4 groups with 10 per group. All mice were intradermally injected with 5×10⁵ B16F10 cells on both sides. After one day of injection, the mice were treated as follows:
Control group, 0.2 ml PBS;
hu-IL-7 protein treatment group, 0.2 ml PBS containing 5 µg hu-IL-7 protein;
hu-IL-25 protein treatment group, 0.2 ml PBS containing 5 µg hu-IL-25 protein;
hu-IL-7 and hu-IL-25 protein combination treatment group, 0.2 ml PBS containing 5
µg of each hu-IL-7 protein and hu-IL-25 protein.

The mice were intraperitoneally injected twice a day, and the tumor area was measured daily using a vernier caliper four days after the start of treatment.

The results are shown in Fig. 4C.

### Example 5

This example utilizes the combination of purified hu-IL-7-HSA and hu-IL-25-HSA to treat human tumors.

Recombinant HSA-IL-7 (as shown in SEQ ID NO: 3) and IL-25-HSA (as shown in SEQ ID NO: 4) were respectively expressed in CHO cells (Chinese hamster ovary cells), and these two purified recombinant proteins were produced in GMP-compliant environment for human tumor therapy.

Administration of all antibiotics was terminated at least one of week prior to treatment and during treatment. Hu-IL-7-HSA and hu-IL-25-HSA were mixed in proportion and then administered intravenously or intraperitoneally or subcutaneously or intramuscularly. The therapeutic dose is 0.0001-100 mg/kg body weight and it was administered once at intervals of average 5-30 days. The tumor size was measured four months later to determine the therapeutic effect.

### Example 6

This example utilizes the combination of purified IgG4m-Fc-IL-7 and IgG4m-Fc-IL-25 to treat human tumors.

Recombinant human IgG4m-Fc-IL-7 (as shown in SEQ ID NO: 5) and IgG4m-Fc-IL-25 (as shown in SEQ ID NO: 6) were respectively expressed in human retinal PER.C6 cells, and these two purified recombinant proteins were produced in GMP-compliant environment for human tumor therapy.

Administration of all antibiotics was terminated at least one of week prior to treatment and during treatment. IgG4m-Fc-IL-7 and IgG4m-Fc-IL-25 were mixed in proportion and then administered intravenously or intraperitoneally or subcutaneously or intramuscularly. The therapeutic dose was 0.0001-10 mg/kg body weight, and it was administered once at intervals of average 5-30 days. The tumor size was measured four months later to determine the therapeutic effect.

### Example 7

This example utilizes autologous T cells co-expressing IL-7 and IL-25 to treat tumors.

The expression cassette co-expressing hu-IL-7, hu-IL-25, and IRES-hu-CD19 with the intracellular domain removed, was cloned into the transposon vector pSBbi bidirectional promoter vector (purchased from Addgene).
50 ml of peripheral blood from a donor was taken, and lymphocytes were isolated by Ficoll-paque. After immunofluorescence staining, CD3⁺PD1⁺CD25⁻ T cells were sorted by flow cytometry.

In addition, T cell populations also can be isolated from Tumor infiltrating lymphocytes isolated from fresh tumor tissues.

Transposon vector (2.5 µg) co-expressing hu-IL-7 and hu-IL-25-IRES-hu-CD19 and SB100X (0.5µg) transposase expression vector were co-transfected into prepared 1×10⁷ T cells by electroporation (Lonza, T cell nucleofection kit). After overnight incubation, cells were incubated with mouse anti-human CD3 antibody-conjugated magnetic beads and mouse anti-human CD28 antibody for 24 to 48 hours, and then incubated with hu-IL-2-containing medium for two days. The transfected T cells were sorted by Biotin-anti-hu-CD19 antibody and magnetic beads of streptavidin and continued to expand to the desired dose.

The prepared T cells were intravenously returned to the donor at a dose of 10⁵-10⁹ cells/kg body weight/time. The T cells was returned once every 5 to 100 days, and the tumor size was measured four months later to determine the therapeutic effect.

### Example 8

This example utilizes cells co-expressing IL-7 and IL-25 or IL-33 encapsulated into sodium alginate microcapsules to treat tumors.

Cells that co-express IL-7 and IL-25 or IL-7 and IL-33 were respectively encapsulated into a multi-layer sodium alginate microencapsulation system (Bhuhbal *et al.,* 2014, Sci. Rep. 4: 6856) as follows.

Lentiviral vectors co-expressing IL-7 and IL-25 or IL-7 and IL-33 were transduced into baby hamster kidney cells (BHK) and cloned and expanded.

The cells were precipitated by centrifugation (final concentration of 6 million/ml) and mixed with medium guluronic acid (G)-sodium alginate (final concentration of 3.4%), and converted into droplets by a 27G needle using an electrostatic bead generator. The droplets were collected in 100 mM CaCl₂ gel solution for 5 minutes. These microspheres were incubated with 0.05% polylysine (PLL). The PLL-coated microcapsules were then co-incubated with 0.34% of medium-G sodium alginate dissolved in calcium-free Krebs-Ringer-Hepes (KRH) solution (120 mM NaCl, KCl 5 mM, 1 mM magnesium chloride, 25 mM sodium bicarbonate, 5.5 mM Hepes, 1 mM glucose, pH 7.2 ± 0.15) to form sodium alginate-poly-L-lysine-sodium alginate (APA) microcapsules. The cells containing the medium G alginate-PLL membrane were then suspended in 2% high G sodium alginate solution. This solution was collected in a 100 mM CaCl₂ gel solution through a 23G needle using another type of droplet generator. These microencapsulated cells were cultured in a cell culture flask with culture medium (DMEM, 10% (v/v) fetal bovine serum, antibiotics and antifungal agent) in a standard tissue culture incubator. The medium was changed every other day. Administration of all antibiotics to the host was terminated at least one of week prior to treatment and during treatment. The microencapsulated cells were then implanted intradermally or intraperitoneally into the animal body at a dose between 1 × 10⁷ and 1 × 10¹² cells, once every 2-12 months, as a combination for cancer therapy.

### Example 9

This example utilizes adenovirus co-expressing hu-IL-7 and hu-IL-25 to treat tumors.

Adenovirus was generated using the adenovirus pAdEasy system (Luo et al 2007, Nat. Protocol, 2, 12360-1247) as follows.

The hu-IL-7 and hu-IL-25 coding sequences were cloned into the shuttle vector of pAdTrack-CMV. After identification by sequencing, the vector was linearized with PmeI, and 0.5 µg of linearized vector was transformed into pAdEasy competent cells by electroporation. The identified cloned plasmid was then transformed into DH10B competent cells, and the recombinant adenovirus plasmid was amplified and purified. 3 µg of adenoviral plasmid was linearized with PacI and transfected into HEK293 adenovirus packaging cells. After 14-20 days, the cells were collected and the adenovirus was extracted by repeated freeze-thaw method. The high titer adenovirus was then amplified and purified stepwise. The titer of the resultant adenovirus was measured and the amount of IL-7 and IL-25 produced by the infected target cells *in vitro* was measured by ELISA. The quality-controlled adenovirus was injected into the patient's tumor with a syringe, and the dose was determined according to the size of the tumor. 10⁸-10¹³ pfu of adenoviruses were injected into each tumor.

### Example 10

This example tests the therapeutic activity against tumors using purified hu-IL-25-hu-IL-7-iRGD recombinant protein.

The recombinant hu-IL-25-hu-IL-7-iRGD recombinant protein (as shown in SEQ ID NO: 30) was expressed in CHO cells (Chinese hamster ovary cells), and the recombinant protein was produced and purified in GMP-compliant environment for tumor therapy.

Administration of all antibiotics was terminated at least one of week prior to treatment and during treatment. The recombinant hu-IL-25-hu-IL-7-iRGD recombinant protein was administered intravenously or intraperitoneally or subcutaneously or intramuscularly or intratumorally, at a therapeutic dose of 0.0001 to 10 mg/kg body weight at average 1-4 times per day, or continuously administered by an insulin pump, and the tumor size was measured four months later to judge the therapeutic effect.

## Claims

1. A pharmaceutical combination for preventing and/or treating a malignant tumor, comprising:
i) at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active portion or a variant thereof; and
ii) at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

2. The pharmaceutical combination according to claim 1, wherein at least one of γc-cytokine comprises IL-2, IL-4, IL-7, IL-9, IL-15 or IL-21; preferably IL- 2, IL-7, IL-9 or IL-15; more preferably IL-7 and IL-2.

3. The pharmaceutical combination according to claim 1 or 2, wherein at least one of γc-cytokine or an active part or variant thereof in i), and/or at least one of IL-25 or IL-33 or an active part or variant thereof in ii) respectively, further comprise one or more modifications selected from the group consisting of: fusion with a human immunoglobulin Fc fragment or a variant thereof; fusion with human serum albumin or a variant thereof; fusion with a tumor-penetrating peptide; fusion with a tumor-targeting single-chain or single-domain antibody; PEGylation; Xtenylation; PASylation; HESylation; and any other suitable chemical modifications.

4. The combination according to any one of claims 1 to 3, wherein the malignant tumor is selected from the group consisting of: fibrosarcoma, mucinous sarcoma, liposarcoma, chondrosarcoma, osteosarcoma, chordoma, angiosarcoma, endothelial sarcoma, lymphangiosarcoma, lymphangial endothelial sarcoma, synovial sarcoma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous gland cancer, papillary carcinoma, papillary adenocarcinoma, cancer, bronchial carcinoma, medullary carcinoma, renal cell carcinoma, liver cancer, cholangiocarcinoma, choriocarcinoma, seminoma, embryonic carcinoma, nephroblastoma, cervical cancer, testicular tumor, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, meningioma, oligodendroglioma, melanoma, neuroblastoma, and retinoblastoma.

5. A kit for preventing and/or treating a malignant tumor, comprising:
a first component: at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active part or variant thereof;
a second component: at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

6. The kit according to claim 5, wherein at least one of yc-cytokine comprises IL-2, IL-4, IL-7, IL-9, IL-15 or IL-21; preferably IL- 2, IL-7, IL-9 or IL-15; more preferably IL-7 and IL-2.

7. The kit according to claim 5 or 6, wherein at least one of γc-cytokine or an active part or variant thereof in i), and/or at least one of IL-25 or IL-33 or an active part or variant thereof in ii), respectively, further comprise one or more modifications selected from the group consisting of: fusion with a human immunoglobulin Fc fragment or a variant thereof; fusion with human serum albumin or a variant thereof; fusion with a tumor-penetrating peptide; fusion with a tumor-targeting single-chain or single-domain antibody; PEGylation; Xtenylation; PASylation; HESylation; and any other suitable chemical modifications.

8. The kit according to any one of claims 5 to 7, wherein the first component and the second component are present in separate pharmaceutical compositions; or the first component and the second component are present in the same pharmaceutical composition.

9. A recombinant protein for preventing and/or treating a malignant tumor, comprising:
at least one of γc-cytokine or an active part or variant thereof; and
at least one of IL-25 or IL-33 or an active part or variant thereof.

10. The recombinant protein according to claim 9, wherein at least one of yc-cytokine comprises IL-2, IL-4, IL-7, IL-9, IL-15 or IL-21; preferably IL- 2, IL-7, IL-9 or IL-15; more preferably IL-7 and IL-2.

11. The recombinant protein according to claim 9, comprising:
an amino acid sequence as set forth in any one of SEQ ID NOs: 1-6 or an active portion thereof, or an amino acid sequence having at least 70%, 80%, 90%, 95% or 99% identity with the amino acid sequence as set forth in any one of SEQ ID NOs: 1-6 or an active portion thereof; and
an amino acid sequence as set forth in SEQ ID NO: 7 or 8 or an active portion thereof, or an amino acid sequence having at least 70%, 80%, 90%, 95% or 99% identity with the amino acid sequence as set forth in SEQ ID NO: 7 or 8 or an active portion thereof.

12. The recombinant protein according to any of claims 9-11, comprising one or more linkers, preferably the peptide linkers that are cleavable *in vivo* or intratumorally.

13. The recombinant protein according to claim 12, wherein the linkers are selected from the group consisting of one or more of the linker as set forth in any one of SEQ ID NOs: 21-29 and 32 and *in vivo* cleavable disulfide bond linker.

14. The recombinant protein according to claim 9, wherein the recombinant protein further comprises one or more modifications selected from the group consisting of:
fusion with a human immunoglobulin Fc fragment or a variant thereof; and fusion with human serum albumin or a variant thereof; fusion with a tumor-penetrating peptide; fusion with a tumor-targeting single-chain or single-domain antibody;
PEGylation; Xtenylation; PASylation; HESylation; and any other suitable chemical modifications.

15. A recombinant nucleic acid molecule for preventing and/or treating a malignant tumor, comprising:
a first nucleotide sequence selected from the group consisting of: the nucleotide sequence as set forth in any one of SEQ ID NOs: 9-14, or a nucleotide sequence having at least 70%, 80%, 90%, 95% or 99% identity with the nucleotide sequence as set forth in any one of SEQ ID NOs: 9-14 or a variant thereof; and/or
a second nucleotide sequence selected from the group consisting of: the nucleotide sequence as set forth in SEQ ID NO: 15 or 16, or a nucleotide sequence having at least 70%, 80%, 90%, 95% or 99% identity with the nucleotide sequence as set forth in SEQ ID NO: 15 or 16 or a variant thereof.

16. A vector for preventing and/or treating a malignant tumor, comprising:
a first nucleotide sequence selected from the group consisting of: the nucleotide sequence as set forth in any one of SEQ ID NOs: 9-14, or a nucleotide sequence having at least 70%, 80%, 90%, 95% or 99% identity with the nucleotide sequence as set forth in any one of SEQ ID NOs: 9-14; and/or
a second nucleotide sequence selected from the group consisting of: the nucleotide sequence as set forth in SEQ ID NO: 15 or 16, or a nucleotide sequence having at least 70%, 80%, 90%, 95% or 99% identity with the nucleotide sequence as set forth in SEQ ID NO: 15 or 16.

17. The vector according to claim 16, wherein the vector is selected from the group consisting of a plasmid and a viral vector, including but not limited to: an adenoviral vector, an adeno-associated viral vector, a retroviral vector, a lentiviral vector, a sleeping beauty or a PiggyBac transposon system, CRISPR/Cas9 knock-in system or any other suitable carriers.

18. The vector according to claim 16 or 17, wherein the first nucleotide sequence and the second nucleotide sequence are present in separate vectors; or wherein the first nucleotide sequence and the second nucleotide sequence are present in the same vector.

19. A cell or a population of cells for preventing and/or treating a malignant tumor, wherein the cell or the population of cells is capable of producing the recombinant protein of any one of claims 9 to 14, or comprises the recombinant nucleic acid molecule of claim 15, or comprises the vector of any one of 16 to 18.

20. The cell or population of cells according to claim 19, wherein the cell is selected from the group consisting of an autologous cell or an allogeneic cell, including but not limited to: dendritic cells, T cells, macrophages, eosinophils, or any other suitable cells.

21. A method for preventing and/or treating a malignant tumor comprising administering to a subject in need thereof a prophylactically and/or therapeutically effective amount of the combination of any one of claims 1 to 4, or the kit of claims 5 to 8, the recombinant protein of any one of claims 9 to 14, or the recombinant nucleic acid molecule of any one of claim 15, or the vector of any one of claims 16 to 18, or the cell or population of cells of claim 19 or 20.

22. The method according to claim 21, when administering the recombinant protein to a subject in need thereof, the dose administered is 0.0001 to 10 mg/kg body weight per time at the interval of 0 to 30 days; or when intratumorally administering the vector such as a virus to a subject in need thereof, the dose administered is 10⁸-10¹³ pfu virus/tumor/time at the interval of 0 to 30 days; or when administering the cell or population of cells to a subject in need thereof, the dose administered is 10⁵-10¹¹ cells/kg body weight/time at the interval of 0 to 100 days.

23. Use of the combination of any one of claims 1 to 4, or the recombinant protein of any one of claims 9 to 14 in the prevention and/or treatment of a malignant tumor, or in preparation of a medicament for prevention and/or treatment of a malignant tumor:
i) at least one of γc-cytokine or an active part or variant thereof, or a vector or a cell or a population of cells capable of producing at least one of γc-cytokine or an active part or variant thereof, or a nucleic acid molecule capable of encoding at least one of γc-cytokine or an active part or variant thereof; and
ii) at least one of IL-25 or IL-33 or an active part or variant thereof, or a vector or a cell or a population of cells capable of producing at least one of IL-25 or IL-33 or an active part or variant thereof, or a nucleic acid molecule capable of encoding at least one of IL-25 or IL-33 or an active part or variant thereof.

24. The use according to claim 23, wherein at least one of γc-cytokine comprises IL-2, IL-4, IL-7, IL-9, IL-15 or IL-21; IL-2, preferably IL-7, IL-9 or IL-15; more preferably IL-7 and IL-2.

25. The use according to claim 23 or 24, wherein at least one of γc-cytokine or an active part or variant thereof in i), and/or at least one of IL-25 or IL-33 or an active part or variant thereof in ii), respectively, further comprise one or more modifications selected from the group consisting of: fusion with a human immunoglobulin Fc fragment or a variant thereof; fusion with human serum albumin or a variant thereof; fusion with a tumor-penetrating peptide; fusion with a tumor-targeting single-chain or single-domain antibody; PEGylation; Xtenylation; PASylation; HESylation; or any other suitable chemical modifications.

26. The use according to any one of claims 23 to 25, wherein the malignant tumor is selected from the group consisting of: fibrosarcoma, mucinous sarcoma, liposarcoma, chondrosarcoma, osteosarcoma, chordoma, angiosarcoma, endothelial sarcoma, lymphangiosarcoma, lymphangial endothelial sarcoma, synovial sarcoma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous gland cancer, papillary carcinoma, papillary adenocarcinoma, cancer, bronchial carcinoma, medullary carcinoma, renal cell carcinoma, liver cancer, cholangiocarcinoma, choriocarcinoma, seminoma, embryonic carcinoma, nephroblastoma, cervical cancer, testicular tumor, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, meningioma, oligodendroglioma, melanoma, neuroblastoma, and retinoblastoma.
